(19) Europäisches Patentamt / European Patent Office / Office européen des brevets

(11) **EP 4 048 356 B1**

(12) **EUROPEAN PATENT SPECIFICATION**

(45) Date of publication and mention
of the grant of the patent:
**21.05.2025  Bulletin 2025/21**

(21) Application number: **20878200.3**

(22) Date of filing: **02.07.2020**

(51) International Patent Classification (IPC):
***A61M 16/00*** *(2006.01)*     ***A61B 5/1455*** *(2006.01)*

(52) Cooperative Patent Classification (CPC):
(C-Sets available)
**A61M 16/026; A61M 16/0051; A61M 16/125;
A61M 16/203; G16H 20/40; G16H 40/63;
G16H 50/50;** A61B 5/14503; A61B 5/14542;
A61B 5/14551; A61M 16/0066; A61M 16/0666;
A61M 16/16; A61M 2016/0039; A61M 2016/1025;
(Cont.)

(86) International application number:
**PCT/IB2020/056237**

(87) International publication number:
**WO 2021/079202 (29.04.2021 Gazette 2021/17)**

(54) **ALARM FOR RESPIRATORY THERAPY SYSTEM**

ALARM FÜR EIN ATEMTHERAPIESYSTEM

ALARME POUR SYSTÈME DE THÉRAPIE RESPIRATOIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **25.10.2019   US 201962925973 P**

(43) Date of publication of application:
**31.08.2022   Bulletin 2022/35**

(73) Proprietor: **Fisher & Paykel Healthcare Limited
East Tamaki
Auckland 2013 (NZ)**

(72) Inventors:
• **GULLEY, Anton Kim
Auckland 2013 (NZ)**
• **HUANG, Yi Lin
Auckland 2013 (NZ)**

(74) Representative: **Gill Jennings & Every LLP
The Broadgate Tower
20 Primrose Street
London EC2A 2ES (GB)**

(56) References cited:
WO-A1-2011/150260     WO-A1-2017/079798
WO-A1-2019/070136     WO-A1-2019/070136
US-A- 5 365 922          US-A1- 2011 290 252
US-A1- 2016 022 952     US-A1- 2016 022 952
US-B1- 7 008 380

(52) Cooperative Patent Classification (CPC): (Cont.)
A61M 2016/103; A61M 2202/0208; A61M 2205/18;
A61M 2205/3334; A61M 2205/3375;
A61M 2205/3561; A61M 2205/3569;
A61M 2205/3584; A61M 2205/505; A61M 2205/52;
A61M 2230/205

C-Sets
A61M 2202/0208, A61M 2202/0007;
A61M 2230/205, A61M 2230/005

## Description

### Field of the invention

The present invention relates to systems for controlling and/or operating a respiratory assistance apparatus.

### Background

Respiratory apparatuses are used in various environments such as hospital, medical facility, residential care, or home environments to deliver a flow of gas to users or patients. A respiratory apparatus, e.g. a flow therapy apparatus, may include an oxygen inlet to allow delivery of supplemental oxygen with the flow of gas, and/or a humidification apparatus to deliver heated and humidified gases. A flow therapy apparatus may allow adjustment and control over characteristics of the gases flow, including flow rate, temperature, gas concentration, such as oxygen concentration, humidity, pressure, etc. WO2019070136A1 discloses a flow therapy apparatus.

### Summary

Disclosed is one invention concerning a respiratory assistance apparatus for providing breathing gases to a patient.

According to the present invention, it is provided respiratory assistance apparatus comprising:

  a flow generator configured to provide a breathing gases to a patient, the breathing gases comprising supplemental oxygen provided from an oxygen source,
  a controller configured to control an oxygen concentration of the breathing gases

    at least one sensor, the at least one sensor configured to provide a measurement indicative of a patient's blood oxygen concentration to the controller,
    wherein the controller is configured to determine a target oxygen concentration of the breathing gases,
    wherein the controller is configured to calculate an estimated future value of the patient's blood oxygen concentration based on:

      a difference between an initial oxygen concentration of the breathing gases and the target oxygen concentration of the breathing gases, and
      the measurement indicative of the patient's blood oxygen concentration.

In some embodiments, the controller is configured to control the oxygen concentration of the breathing gases based on the target oxygen concentration.

In some embodiments, the estimated future value of the patient's blood oxygen concentration is further based on a time from when the controller controls the oxygen concentration of the breathing gases to the target oxygen concentration.

In some embodiments, the controller is configured to receive an input, and based on the input determine an target oxygen concentration of the breathing gases.

In some embodiments, the input comprises a target oxygen concentration range comprising an upper target oxygen concentration, and a lower target oxygen concentration.

In some embodiments a humidifier, the controller, the flow generator and sensors of the respiratory assistance are disposed in a single housing.

In some embodiments, the apparatus comprises at least one gases composition sensor, the gases composition sensor configured to provide a measurement indicative of the oxygen concentration of the breathing gases to the controller.

In some embodiments, the at least one gases composition sensor is an ultrasonic sensor.

In some embodiments, the controller is configured to compare said estimated future value of the patient's blood oxygen concentration with a blood oxygen concentration alarm threshold and/or a blood oxygen concentration alarm range, and generate an alarm output if the estimated future value of the patient's blood oxygen concentration is not within said blood oxygen concentration alarm range.

In some embodiments, the blood oxygen concentration alarm range comprises an upper blood oxygen concentration alarm threshold, and a lower blood oxygen concentration alarm threshold.

In some embodiments, the blood oxygen concentration alarm threshold is determined based on the time from when the controller controls the oxygen concentration of the breathing gases to the target oxygen concentration.

In some embodiments, the alarm threshold may be determined based on an amount of a change in target oxygen concentration.

In some embodiments, the alarm threshold may be determined or target oxygen concentration

In some embodiments, the alarm threshold may be determined current oxygen concentration of gases.

In some embodiments, the alarm threshold is based on the target blood oxygen concentration.

In some embodiments, the controller is configured to generate an alarm output based on the estimated future value of the patient's blood oxygen concentration and a comparison with to a blood oxygen concentration alarm threshold.

In some embodiments, the controller is configured to generate said alarm output if the estimated future value of the patient's blood oxygen concentration is above or below the blood oxygen concentration threshold.

In some embodiments, the alarm threshold is based on the time from when the controller controls the oxygen concentration of the breathing gases to the target oxygen concentration.

In some embodiments, the input comprises a target blood oxygen concentration, and the blood oxygen concentration alarm range is based on the target blood oxygen concentration.

In some embodiments, the blood oxygen concentration threshold and/or blood oxygen concentration alarm range are based on the difference between the target oxygen concentration and the upper target oxygen concentration.

In some embodiments, the respiratory assistance apparatus may comprise at least one valve, wherein the controller is configured to control the valve to control the oxygen concentration of the breathing gases by varying the amount of oxygen provided from an oxygen source to the breathing gas.

In some embodiments, respiratory assistance apparatus may be connected to an oxygen supply, wherein the controller is configured to control the flow of gases from the oxygen supply to control the oxygen concentration of the breathing gases.

In some embodiments the controller is configured to control the oxygen concentration of the breathing gases based on the target oxygen concentration and a measured oxygen concentration of the breathing gases.

In some embodiments, the measurement indicative of the patient's blood oxygen concentration may be determined over a predetermined time.

In some embodiments, the controller is configured to generate an alarm output based on the estimated future value of the patient's blood oxygen concentration.

In some embodiments, the respiratory assistance apparatus (or an associated device) is configured to display the alarm output, or information associated with the alarm output.

In some embodiments, the respiratory assistance apparatus (or an associated device) is configured to display the estimated future value of the patient's blood oxygen concentration.

In some embodiments, the alarm output may be transmitted to one or more servers, or a patient monitoring unit, and/or a nurse monitoring station, in communication with the respiratory assistance apparatus.

In a comparative example there is provided a respiratory assistance apparatus comprising:

a flow generator configured to provide a breathing gas to a patient, the breathing gas comprising supplemental oxygen provided from an oxygen source,

at least one sensor, the at least one sensor configured to provide a measurement indicative of the patient's blood oxygen concentration to the controller,

a controller, the controller configured to receive an input, the input comprising a target oxygen concentration range comprising an upper target oxygen concentration, and/or a lower target oxygen concentration,

a controller, the controller configured to control an oxygen concentration of the breathing gases to a target oxygen concentration, where the target oxygen concentration is within said target oxygen concentration range comprising an upper target oxygen concentration and a lower target oxygen concentration,

wherein the controller is configured to:

calculate an estimated maximum future value of a patient's blood oxygen concentration for the target oxygen concentration range based on the measurement indicative of a patient blood oxygen concentration and a difference between the target oxygen concentration and the upper target oxygen concentration or

calculate an estimated minimum future value of a patient's blood oxygen concentration for the target oxygen concentration range based on the measurement indicative of a patient blood oxygen concentration, and a difference between the target oxygen concentration and the lower target oxygen concentration.

In some embodiments, the controller is configured to control an oxygen concentration of the breathing gases from an initial oxygen concentration to a target oxygen concentration.

In some embodiments, the initial oxygen concentration is an oxygen concentration before the controller controls the oxygen concentration of the breathing gases to the target oxygen concentration.

In some embodiments, the respiratory assistance apparatus is configured to receive an input indicative of the target oxygen concentration range.

In some embodiments, the target oxygen concentration range is input via a user interface.

In some embodiments, the estimated maximum future value and/or the estimated minimum future value is based on an estimated oxygen efficiency.

In some embodiments, the estimated maximum future value and/or the estimated minimum future value is updated in real time.

In some embodiments, the maximum or minimum future value is after an effect of a change of oxygen concentration has been realised on patient blood oxygen.

In some embodiments, the controller is configured to display the estimated minimum future value of a patient's blood oxygen and/or the estimated minimum future value of a patient's blood oxygen concentration.

In some embodiments, the controller is configured to generate an alarm output.

In some embodiments, the alarm output may be transmitted to one or more servers, or a patient monitoring unit, and/or a nurse monitoring station, in communication with the respiratory assistance apparatus.

In some embodiments, the alarm output comprises at least one alarm parameter.

In some embodiments, the controller is configured to generate the alarm output based on the estimated future value of the patient's blood oxygen concentration, and/or the estimated maximum or minimum future value of a patient's blood oxygen concentration

In some embodiments, the controller is configured to compare said estimated maximum future value of a patient's blood oxygen concentration with the upper target blood oxygen concentration, and generate the alarm output if the estimated maximum future value of a patient's blood oxygen concentration is less than the upper target blood oxygen concentration.

In some embodiments, an alarm parameter of the or an alarm output is based on the magnitude of the difference between the estimated maximum future value of a patient's blood oxygen concentration with the upper target blood oxygen concentration.

In some embodiments, the controller is configured to compare said estimated minimum future value of a patient's blood oxygen concentration with the lower target blood oxygen concentration, and generate an alarm output if the estimated minimum future value of a patient's blood oxygen concentration is less than the lower target blood oxygen concentration.

In some embodiments, the or an alarm parameter of the alarm output is based on the magnitude of the difference between the estimated minimum future value of a patient's blood oxygen concentration with the lower target blood oxygen concentration.

In some embodiments, the alarm parameter is the alarm time, or the alarm intensity.

In some embodiments, the alarm output, and/or associated data is provided to a display.

In some embodiments, the alarm output is configured to present a message on a display.

In some embodiments, the alarm output is configured to generate a sound, or provide an indication.

In another comparative example there is provided a respiratory assistance apparatus comprising

a flow generator configured to provide a breathing gas to a patient, the breathing gas comprising supplemental oxygen provided from an oxygen source,

at least one sensor, the at least one sensor configured to provide a measurement indicative of the patient's blood oxygen concentration to the controller,

wherein during operation of the apparatus the controller is configured to store breathing gases oxygen concentration data, wherein the stored breathing gases oxygen concentration data comprises: a plurality of oxygen concentrations of the breathing gases each oxygen concentration of the breathing gases having an associated timestamp ,

wherein the controller is configured to calculate an estimated future value of the patient's blood oxygen concentration based on:

the stored breathing gases oxygen concentration data, wherein oxygen concentrations of the breathing gases are weighted based on the associated timestamp, and

the measurement indicative of the patient's blood oxygen concentration.

In some embodiments, the controller is configured to calculate an estimated future value of the patient's blood oxygen concentration based on:

an estimated oxygen efficiency ratio for the patient,

a haemoglobin saturation function.

In some embodiments, the controller is configured to generate an alarm output based on the estimated future value of the patient's blood oxygen concentration.

In some embodiments, the respiratory assistance apparatus (or an associated device) is configured to display the alarm output, or information associated with the alarm output.

In some embodiments, the respiratory assistance apparatus (or an associated device) is configured to display the estimated future value of the patient's blood oxygen concentration.

In some embodiments, the alarm output may be transmitted to one or more servers, or a patient monitoring unit, and/or a nurse monitoring station, in communication with the respiratory assistance apparatus.

In another comparative example there is provided a respiratory assistance apparatus comprising

a flow generator configured to provide a breathing gas to a patient, the breathing gas comprising supplemental oxygen provided from an oxygen source,

at least one sensor, the at least one sensor configured to provide a measurement indicative of the patient's blood oxygen concentration to the controller,

wherein during operation of the apparatus the controller is configured to store breathing gases oxygen concentration data, wherein the stored breathing gases oxygen concentration data comprises one or more oxygen concentrations of the breathing gases each oxygen concentrations of the breathing gases having an associated timestamp,

wherein the controller is configured to calculate an estimated maximum future value of the patient's blood oxygen concentration based on:

the stored breathing gases oxygen concentration data, wherein oxygen concentrations of the breathing gases are weighted based on the associated timestamp, and
the measurement indicative of the patient's blood oxygen concentration
a difference between the current oxygen concentration of the breathing gases and a upper target oxygen concentration.

In some embodiments, the controller is configured to calculate an estimated maximum future value of the patient's blood oxygen concentration based on:

an estimated oxygen efficiency ratio for the patient,

a haemoglobin saturation function.

In another comparative example there is provided respiratory assistance apparatus comprising

a flow generator configured to provide a breathing gas to a patient, the breathing gas comprising supplemental oxygen provided from an oxygen source,

at least one sensor, the at least one sensor configured to provide a measurement indicative of the patient's blood oxygen concentration to the controller,

wherein during operation of the apparatus the controller is configured to store breathing gases oxygen concentration data, wherein the stored breathing gases oxygen concentration data comprises one or more oxygen concentrations of the breathing gases each oxygen concentrations of the breathing gases having an associated timestamp,

wherein the controller is configured to calculate an estimated minimum future value of the patient's blood oxygen concentration based on:

the stored breathing gases oxygen concentration data, wherein oxygen concentrations of the breathing gases are weighted based on the associated timestamp, and

the measurement indicative of the patient's blood oxygen concentration

a difference between the oxygen concentration of the breathing gases at the current estimation phase and a lower target oxygen concentration.

In some embodiments, the controller is configured to calculate an estimated minimum future value of the patient's blood oxygen concentration based on:

an estimated oxygen efficiency ratio for the patient,

a haemoglobin saturation function.

In some embodiments, the associated timestamp comprises a time when the associated oxygen concentration of the breathing gases was recorded, and/or measured and/or stored.

In some embodiments, the associated timestamp comprises a time elapsed since the associated oxygen concentration of the breathing gases was recorded, and/or measured and/or stored.

In some embodiments, the controller is configured to determine a series of changes to oxygen concentration of the breathing gases in the stored breathing gases oxygen concentration data.

In some embodiments, the oxygen concentration of the breathing gases is controlled to a target oxygen concentration of the breathing gases.

In some embodiments, the oxygen concentration of the breathing gases is a measured oxygen concentration of the breathing gases.

In some embodiments, the apparatus comprises at least one gases composition sensor, the gases composition sensor configured to provide a measurement indicative of the oxygen concentration of the breathing gases to the controller.

In some embodiments, the controller is configured to updated the stored the oxygen concentration of the breathing gases concentration data at regular time intervals, or irregular time intervals.

In some embodiments, the controller is configured to update the stored oxygen concentration of the breathing gases when a target oxygen concentration of the breathing gases is updated by the controller.

In some embodiments, the weighting based on the associated timestamp is based on a decay function.

In some embodiments, the stored oxygen concentration data is transmitted to one or more servers, or a patient monitoring unit, and/or a nurse monitoring station, in communication with the respiratory assistance apparatus.

In another comparative example there is provided respiratory assistance apparatus comprising

a flow generator configured to provide a breathing gas to a patient, the breathing gas comprising supplemental oxygen provided from an oxygen source,

at least one sensor, the at least one sensor configured to provide a measurement indicative of the patient's blood oxygen concentration to the controller,

wherein the controller is configured to maintain a sum of changes of the breathing gases oxygen concentration,

wherein the controller is configured to execute an estimation update phase, the estimation update phase comprising:

applying a decay function to the sum of changes of the oxygen concentration of the breathing gases,

calculating a difference between the oxygen concentration of the breathing gases at the current estimation update phase, and the oxygen concentration of the breathing gases at the previous estimation update phase,

adding the difference to the sum of changes to the oxygen concentration of the breathing gases

wherein the controller is configured to calculate an estimated future value of the patient's blood oxygen concentration based on:

the sum of changes of the oxygen concentration of the breathing gases, and

the measurement indicative of the patient's blood oxygen concentration.

The controller is configured to calculate an estimated future value of the patient's blood oxygen concentration based on:

an estimated oxygen efficiency ratio for the patient,

a haemoglobin saturation function.

In another comparative example there is provided respiratory assistance apparatus comprising

a flow generator configured to provide a breathing gas to a patient, the breathing gas comprising supplemental oxygen provided from an oxygen source,

at least one sensor, the at least one sensor configured to provide a measurement indicative of the patient's blood oxygen concentration to the controller,

wherein the controller is configured to maintain a sum of changes of the breathing gases oxygen concentration,

wherein the controller is configured to execute an estimation update phase, the estimation update phase comprising:

applying a decay function to the sum of changes of the oxygen concentration of the breathing gases,

calculating a difference between the oxygen concentration of the breathing gases at the current estimation update phase, and the oxygen concentration of the breathing gases at the previous estimation update phase,

adding the difference to the sum of changes to the oxygen concentration of the breathing gases

wherein the controller is configured to calculate an estimated maximum future value of the patient's blood oxygen concentration based on:

the sum of changes of the oxygen concentration of the breathing gases , and

the measurement indicative of the patient's blood oxygen concentration

a difference between the oxygen concentration of the breathing gases at the current estimation phase and a upper target oxygen concentration.

In some embodiments, the controller is configured to calculate an estimated maximum future value of the patient's blood oxygen concentration based on:

an estimated oxygen efficiency ratio for the patient,

a haemoglobin saturation function.

In another comparative example there is provided a respiratory assistance apparatus comprising

a flow generator configured to provide a breathing gas to a patient, the breathing gas comprising supplemental oxygen provided from an oxygen source,

at least one sensor, the at least one sensor configured to provide a measurement indicative of the patient's blood oxygen concentration to the controller,

wherein the controller is configured to maintain a sum of changes of the breathing gases oxygen concentration,

wherein the controller is configured to execute an estimation update phase, the estimation update phase comprising:

applying a decay function to the sum of changes of the oxygen concentration of the breathing gases,

calculating a difference between the oxygen concentration of the breathing gases at the current estimation update phase, and the oxygen concentration of the breathing gases at the previous estimation update phase,

adding the difference to the sum of changes to the oxygen concentration of the breathing gases

wherein the controller is configured to calculate an estimated minimum future value of the patient's blood oxygen concentration based on:

the sum of changes of the oxygen concentration of the breathing gases , and

the measurement indicative of the patient's blood oxygen concentration

a difference between the oxygen concentration of the breathing gases at the current estimation phase and a lower target oxygen concentration.

In some embodiments, the controller is configured to calculate an estimated minimum future value of the patient's blood oxygen concentration based on:

an estimated oxygen efficiency ratio for the patient,

a haemoglobin saturation function.

In some embodiments, the controller is configured to execute the estimation update phase at regular time intervals, or irregular time intervals.

In some embodiments, the controller is configured to execute the estimation update in real time.

In some embodiments, the controller is configured to execute the estimation update phase periodically.

In some embodiments, the controller is configured to execute the estimation update phase when a target oxygen concentration of the breathing gases is updated by the controller.

In some embodiments, the controller is configured to control the oxygen concentration of the breathing gases to a target oxygen concentration,

In some embodiments, the oxygen concentration of the breathing gases is controlled to a target oxygen concentration of the breathing gases.

In some embodiments, the oxygen concentration of the breathing gases is a measured oxygen concentration of the breathing gases.

In some embodiments, the apparatus comprises at least one gases composition sensor, the gases composition sensor configured to provide a measurement indicative of the oxygen concentration of the breathing gases to the controller.

In some embodiments, the controller is configured to update a target oxygen concentration of the breathing gases, and control an oxygen concentration of the breathing gases from an initial oxygen concentration to the target oxygen concentration.

In some embodiments, the target oxygen concentration is provided by an oxygen concentration controller.

In some embodiments, the or a input further comprises the target blood oxygen concentration.

In some embodiments, the or a target blood oxygen concentration is a range.

In some embodiments, the target blood oxygen concentration comprises an upper target blood oxygen concentration and/or a lower target blood oxygen concentration.

In some embodiments, the controller is configured to determine the target oxygen concentration based on a mid point of target blood oxygen concentration range.

In some embodiments, the controller is configured to control the target blood oxygen concentration to be within the upper target blood oxygen concentration and the lower target blood oxygen concentration.

In some embodiments, the controller is configured to vary the target oxygen concentration to control the patient blood oxygen concentration to the target blood oxygen concentration.

In some embodiments, the controller is configured to vary the amount of supplemental oxygen provided from an oxygen source to the breathing gas to control the oxygen concentration of the breathing gases.

In some embodiments, the apparatus comprises at least one valving arrangement.

In some embodiments, the valving arrangement is in fluid communication with the blower

In some embodiments, the valving arrangement may be controllable to adjust the amount of oxygen introduced into the gases flow.

In some embodiments, the controller is configured to generate an alarm output.

In some embodiments, the alarm output may be transmitted to one or more servers, or a patient monitoring unit, and/or a nurse monitoring station, in communication with the respiratory assistance apparatus.

In some embodiments, the alarm output comprises at least one alarm parameter.

In some embodiments, the controller is configured to generate the alarm output based on the estimated future value of the patient's blood oxygen concentration, and/or the estimated maximum or minimum future value of a patient's blood oxygen concentration

In some embodiments, the controller is configured to compare said estimated maximum future value of a patient's blood oxygen concentration with the upper target blood oxygen concentration, and generate the alarm output if the estimated maximum future value of a patient's blood oxygen concentration is less than the upper target blood oxygen concentration.

In some embodiments, an alarm parameter of the or an alarm output is based on the magnitude of the difference between the estimated maximum future value of a patient's blood oxygen concentration with the upper target blood oxygen concentration.

In some embodiments, the controller is configured to compare said estimated minimum future value of a patient's blood oxygen concentration with the lower target blood oxygen concentration, and generate an alarm output if the estimated minimum future value of a patient's blood oxygen concentration is less than the lower target blood oxygen concentration.

In some embodiments, the or an alarm parameter of the alarm output is based on the magnitude of the difference between the estimated minimum future value of a patient's blood oxygen concentration with the lower target blood oxygen concentration.

In some embodiments, the alarm parameter is the alarm time, or the alarm intensity.

In some embodiments, the alarm output, and/or associated data is provided to a display.

In some embodiments, the alarm output is configured to present a message on a display.

In some embodiments, the alarm output is configured to generate a sound, or provide an indication.

In some embodiments, the or an at least one sensor is in electronic communication with the controller.

In some embodiments, the least one sensor is a pulse oximeter, or an arterial oxygen sensor.

In some embodiments, the respiratory assistance apparatus further comprises a gas composition sensor.

In some embodiments, the gas composition sensor is in electronic communication with the controller.

In some embodiments, the gas composition sensor is an oxygen concentration sensor configured to measure the oxygen concentration of the breathing gas.

In some embodiments, the at least one gases composition sensor provides a signal indicative of the initial oxygen concentration of the breathing gases.

In some embodiments, the controller is configured to receive an oxygen concentration signal indicative of the oxygen concentration of the breathing gas from the oxygen concentration sensor.

In some embodiments, the controller is configured to control the oxygen concentration of the breathing gases based on the oxygen concentration signal from the oxygen concentration sensor.

In some embodiments, the controller comprises one or more processors, wherein the processors are configured with computer readable instructions.

In some embodiments, the controller comprises at least one memory element, wherein the memory element is configured to store said computer readable instructions.

In some embodiments, the memory element is non-transitory.

In some embodiments, the flow generator is or comprises a blower module, wherein the blower module comprises at least one blower configured to generate said flow of gases.

In some embodiments, the respiratory assistance apparatus comprises at least one display, configured to display to the or an alarm output.

In some embodiments, the display comprises at least one screen.

In some embodiments, the respiratory assistance apparatus comprises at least one sound generating device configured to output an audible sound.

In another system comprising the respiratory assistance apparatus of any one of the above comparative examples or aspect, a conduit and a user interface.

In another comparative example there is provided a method of estimating a future value of a patients' blood oxygen concentration, the method comprising:

providing the patient with a breathing gases having an initial target oxygen concentration,

measuring a patient's blood oxygen concentration,

providing the patient with a breathing gases having an target oxygen concentration,

calculating a future value of the patient's blood oxygen concentration, based on the patient's blood oxygen concentration and a difference between an initial target oxygen concentration, and the target oxygen concentration.

In another comparative example there is provided a method of estimating a maximum future value of a patient's blood oxygen concentration for a target oxygen concentration range, the method comprising:

measuring a patient's blood oxygen concentration,

providing the patient with a breathing gases having an oxygen concentration,

calculating a maximum future value of a patient's blood oxygen concentration based on the patient's blood oxygen concentration, and a difference between an upper target oxygen concentration of the target oxygen concentration range and the oxygen concentration of the breathing gases.

In another comparative example there is provided a method of estimating a minimum future value of a patient's blood oxygen concentration for a target oxygen concentration range, the method comprising

measuring a patient's blood oxygen concentration,

providing the patient with a breathing gases having an oxygen concentration,

calculating a minimum future value of a patient's blood oxygen concentration based on the patient's blood oxygen concentration, and a difference between an lower target oxygen concentration of the target oxygen concentration range and the oxygen concentration of the breathing gases.

In another comparative example there is provided a method of estimating a future value of a patients' blood oxygen concentration, the method comprising:

providing the patient with a breathing gases,

during operation of the apparatus storing breathing gases oxygen concentration data, wherein the stored breathing gases oxygen concentration data comprises one or more oxygen concentrations of the breathing gases each oxygen concentrations of the breathing gases having an associated timestamp,

measuring a patient's blood oxygen concentration,

calculating an estimated future value of the patient's blood oxygen concentration based on:

the stored breathing gases oxygen concentration data, wherein the oxygen concentrations of the breathing gases are is weighted based on the associated timestamp, and

the measurement indicative of the patient's blood oxygen concentration.

In another comparative example there is provided a method of estimating an estimated maximum future value of the patient's blood oxygen concentration, the method comprising:

providing the patient with a breathing gases,

during operation of the apparatus storing breathing gases oxygen concentration data, wherein the stored breathing gases oxygen concentration data comprises one or more oxygen concentrations of the breathing gases each oxygen concentrations of the breathing gases having an associated timestamp,

measuring a patient's blood oxygen concentration,

calculating an estimated maximum future value of the patient's blood oxygen concentration based on:

the stored breathing gases oxygen concentration data, wherein the oxygen concentrations of the breathing gases are weighted based on the associated timestamp, and
the measurement indicative of the patient's blood oxygen concentration
a difference between the current oxygen concentration of the breathing gases and a upper target oxygen concentration.

In another comparative example there is provided a method of estimating an estimated minimum future value of the patient's blood oxygen concentration, the method comprising:

providing the patient with a breathing gases,

during operation of the apparatus storing breathing gases oxygen concentration data, wherein the stored breathing gases oxygen concentration data comprises one or more oxygen concentrations of the breathing gases each oxygen concentrations of the breathing gases having an associated timestamp,

measuring a patient's blood oxygen concentration,

calculating an estimated minimum future value of the patient's blood oxygen concentration based on:

the stored breathing gases oxygen concentration data, wherein the oxygen concentrations of the breathing gases are weighted based on the associated timestamp, and
the measurement indicative of the patient's blood oxygen concentration
a difference between the oxygen concentration of the breathing gases at the current estimation phase and a lower target oxygen concentration.

In another comparative example there is provided a method of estimating a future value of a patients' blood oxygen concentration, the method comprising:

providing the patient with a breathing gases,

maintaining a sum of changes of the breathing gases oxygen concentration,

executing an estimation update phase, the estimation update phase comprising:

applying a decay function to the sum of changes of the oxygen concentration of the breathing gases,

calculating a difference between the oxygen concentration of the breathing gases at the current estimation update phase, and the oxygen concentration of the breathing gases at the previous estimation update phase,

adding the difference to the sum of changes to the oxygen concentration of the breathing gases.

In some embodiments, the controller is configured to calculate an estimated future value of the patient's blood oxygen concentration based on:

the sum of changes of the oxygen concentration of the breathing gases, and

the measurement indicative of the patient's blood oxygen concentration.

In some embodiments, controller is configured to calculate an estimated maximum future value of the patient's blood oxygen concentration based on:

the sum of changes of the oxygen concentration of the breathing gases , and

the measurement indicative of the patient's blood oxygen concentration a difference between the oxygen concentration of the breathing gases at the current estimation phase and a upper target oxygen concentration.

In some embodiments, the controller is configured to calculate an estimated minimum future value of the patient's blood oxygen concentration based on:

the sum of changes of the oxygen concentration of the breathing gases , and

the measurement indicative of the patient's blood oxygen concentration

a difference between the oxygen concentration of the breathing gases at the current estimation phase and a lower target oxygen concentration.

In another comparative example there is provided a system comprising a respiratory assistance apparatus, and an auxiliary device, wherein the estimated maximum future value, and/or the estimated minimum future value, and/or the estimated future value is calculated on the auxiliary device.

In another comparative example there is provided a system comprising a respiratory assistance apparatus, and an auxiliary device, wherein the controller is located at least in part on the auxiliary device.

In some embodiments, the respiratory apparatus provides sensor outputs and/or stored data to the auxiliary device.

In some embodiments, the or an alarm output may be provided on the respiratory assistance apparatus.

In some embodiments, the or an alarm output may be provided on the auxiliary device.

In a another comparative example there is provided a respiratory assistance apparatus used for providing the method of any of the above comparative examples or aspect.

In a another comparative example there is provided use of a respiratory assistance apparatus comprising for providing the method of any of the above comparative examples or aspect.

In a another comparative example there is provided a respiratory assistance apparatus or use of a flow therapy apparatus of any of the above comparative example or aspect.

It is intended that reference to a range of numbers disclosed herein (for example, 1 to 10) also incorporates reference to all rational numbers within that range (for example, 1, 1.1, 2, 3, 3.9, 4, 5, 6, 6.5, 7, 8, 9 and 10) and also any range of rational numbers within that range (for example, 2 to 8, 1.5 to 5.5 and 3.1 to 4.7).

Embodiments described herein can also be said broadly to relate to the parts, elements and features referred to or indicated in the specification of the application, individually or collectively, and any or all combinations of any two or more of said parts, elements or features, and where specific integers are mentioned herein which have known equivalents in the art to which this invention relates, such known equivalents are deemed to be incorporated herein as if individually set forth.

In this specification, where reference has been made to external sources of information, including patent specifications and other documents, this is generally for the purpose of providing a context for discussing the features of the present invention. Unless stated otherwise, reference to such sources of information is not to be construed, in any jurisdiction, as an admission that such sources of information are prior art or form part of the common general knowledge in the art.

The term "comprising" as used in this specification means "consisting at least in part of". When interpreting statements in this specification which include that term, the features, prefaced by that term in each statement, all need to be present but other features can also be present. Related terms such as "comprise" and "comprised" are to be interpreted in the same manner.

These and other features and aspect and comparative examples are described in detail below with reference to the accompanying drawings.

## Brief description of the drawings

The technology will now be described by way of example only and with reference to the drawings in which:

Figure 1A shows in diagrammatic form a flow therapy apparatus.

Figure 1B illustrates a sensing circuit board, including a flow rate sensor that may be used in a flow therapy apparatus.

Figures 1C-1D illustrate schematic diagrams of various ultrasonic transducer configurations for the sensor assembly using cross-flow beams.

Figures 1E-1F illustrate schematic diagrams of various ultrasonic transducer configurations for the sensor assembly using along-flow beams.

Figures 2A 2B, and Figure 3 illustrate graphs showing operation of a respiratory device.

Figure 4A to 4D illustrate a process for estimating a future value of a patient's blood oxygen.

Figures 5A and 5B illustrate alarm conditions for a respiratory device.

Figure 6 illustrates a process for estimating a maximum or minimum future value of a patient's blood oxygen.

Figure 7 illustrates a graph showing operation of a respiratory device.

Figures 8A and 8B illustrate graphs showing operation of a respiratory device.

Figures 9A and 9B illustrate a process for estimating a maximum or minimum future value of a patient's blood oxygen.

Figure 10 illustrates a graph showing operation of a respiratory device.

Figure 10A illustrates a process for estimating oxygen efficiency of a patient.

Figure 11 is a first underside perspective view of the main housing of the flow therapy apparatus showing a recess inside the housing for the motor and/or sensor module sub-assembly.

Figure 12 is a second underside perspective view of the main housing of the flow therapy apparatus showing the recess for the motor and/or sensor module sub-assembly.

Figure 13 is a perspective view of the motor and/or sensor subassembly, underside of the main housing, and fixed elbow of the flow therapy apparatus.

Figure 14 is an exploded perspective view of components of the motor and/or sensor sub-assembly schematically showing by way of an arrow the gas flow path through the sub-assembly.

Figure 15 is an underside view of a cover and sensing PCB of the motor and/or sensor sub-assembly showing the position of sensors.

Figure 16 is a rear perspective view of the flow therapy apparatus sectioned adjacent to the rear edge of the flow therapy apparatus, showing the arrangement of a portion of the main housing that provides the recess for receipt of the motor and/or sensor sub-assembly.

Figure 17A is a left front perspective view of the flow therapy apparatus.

Figure 17B is a left front perspective view of the flow therapy apparatus.

Figure 18 is a left front perspective partial cutaway view showing the valve module and the filter module.

Figure 19 is a schematic gas flow path diagram for the filter module and the valve module, with the solid line arrows representing the flow of oxygen (or another gas), and the dashed line arrows representing the flow of ambient air.

Figure 20 is a sectional view showing the gas flow path through the filter module and the valve module.

Figure 21 is a rear side overhead perspective view of a first configuration valve module.

Figure 22 is a rear side overhead perspective view showing the gas flow paths through the first configuration valve module, with the solid line arrows representing the flow of oxygen (or another gas), and the dashed line arrow representing the flow of ambient air.

Figure 23 is a sectional view through the first configuration valve module.

Figure 24 is a sectional view showing the coupling of, and gas flow path through, the valve and valve manifold of the first configuration valve module.

Figure 25 is a diagram of a respiratory assistance apparatus and an auxiliary device.

## Detailed description

Patients suffering from various health conditions and diseases can benefit from respiratory therapy. In at least one form, the respiratory therapy may be oxygen therapy. For example, a patient suffering from chronic obstructive pulmonary disease (COPD), pneumonia, asthma, bronchopulmonary dysplasia, heart failure, cystic fibrosis, sleep apnea, lung disease, trauma to the respiratory system, acute respiratory distress and/or other conditions or diseases can benefit from respiratory therapy. Similarly, patients receiving pre- and post- operative oxygen delivery can also benefit from respiratory therapy. A common way of treating such problems is by supplying the patient with breathing gas that contains supplemental oxygen. In at least one configuration, the breathing gas can be supplied with supplemental oxygen of a controlled concentration. This supplemental oxygen helps to prevent the patient's blood oxygen concentration (for example, blood oxygen saturation (SpO2)) from dropping too low (e.g., below about 90%).

However, when providing respiratory therapy to a patient such as by providing supplemental oxygen, changes to the blood oxygen concentration of the patient may lag changes to the provided oxygen concentration. That is, any change in blood oxygen concentration may take a significant time to occur from when a change in the provided oxygen concentration is made. In other words, when providing respiratory therapy such as supplemental oxygen therapy to a patient, there may be a significant delay between a change in the provided oxygen concentration and the resulting change in patient blood oxygen concentration. Therefore, it can be difficult for a user to determine whether a change in provided oxygen concentration will meet the desired therapy target in terms of patient blood oxygen concentration until significant time has passed. During this time, the patient may not be provided with sufficient respiratory therapy.

A respiratory therapy apparatus 10 may therefore be provided that estimates the effect of a change in oxygen concentration of the breathing gases on a patient blood oxygen concentration.

One benefit is that a user can quickly see the effect of the change in oxygen concentration of the breathing gases on patient blood oxygen concentration and determine whether this meets their therapy target (for example a desired blood oxygen concentration).

Another benefit is that the respiratory therapy apparatus 10 may alarm based on the estimated future value of the patient's blood oxygen concentration. For example, the respiratory therapy apparatus 10 may alarm when the change in the provided oxygen concentration of the breathing gases is estimated to be unlikely to provide the desired change in patient blood oxygen concentration.

When a user typically a therapist, nurse, doctor, or other health practitioner, sets a target blood oxygen concentration, they may also set a target oxygen concentration range of the breathing gases. The respiratory therapy apparatus 10 will then attempt to control the patient blood oxygen concentration by varying the provided oxygen concentration of the breathing gases while ensuring the oxygen concentration of the breathing gases is within the target oxygen concentration range.

However, in some cases, the respiratory therapy apparatus 10 will be unable to reach the target blood oxygen concentration when providing breathing gases with a composition within the target oxygen concentration range. Again, in this case, the patient may not be receiving sufficient therapy.

Additionally, as described above, changes to the blood oxygen concentration of the patient may lag changes to the provided oxygen concentration. Therefore, it may be some time before the user realises that the patient is not receiving sufficient therapy.

A respiratory therapy apparatus 10 may therefore be provided that estimates the maximum or minimum future blood oxygen concentration of a patient corresponding with the target oxygen range.

It will be appreciated the terms respiratory assistance apparatus, respiratory therapy apparatus and flow therapy apparatus may be used interchangeably.

The apparatus may then generate an alarm output to notify the user that the apparatus will be unable to reach the target blood oxygen concentration when providing breathing gases with a composition within the target oxygen concentration range. The user can then review the patient and, optionally, modify the target oxygen concentration range.

In some embodiments, the apparatus may determine an updated target oxygen concentration range if the apparatus detects it will be unable to reach the target blood oxygen concentration with the target oxygen concentration range.

The estimation of the maximum or minimum future blood oxygen concentration may provide an early indication to the user the patient is not receiving sufficient therapy.

Blood oxygen concentration may be a peripheral capillary oxygen saturation (SpO2), or a partial pressure of oxygen (PaO2).

Oxygen concentration of breathing gases may be a fraction of inspired oxygen (FiO2), for example the oxygen percentage of the breathing gas inhaled by the patient, or a fraction of delivered oxygen (FdO2), for example the oxygen percentage of the breathing gas delivered to the patient.

Physiologically, oxygen provided to a patient (for example as a fraction of inspired oxygen (FiO2)), will be via the lungs transferred to the blood of a patient (for example as a partial pressure of oxygen (PaO2))

The oxygen in a patient's blood (for example as a partial pressure of oxygen (PaO2)) may be converted to a peripheral capillary oxygen saturation (SpO2) using a haemoglobin saturation function.

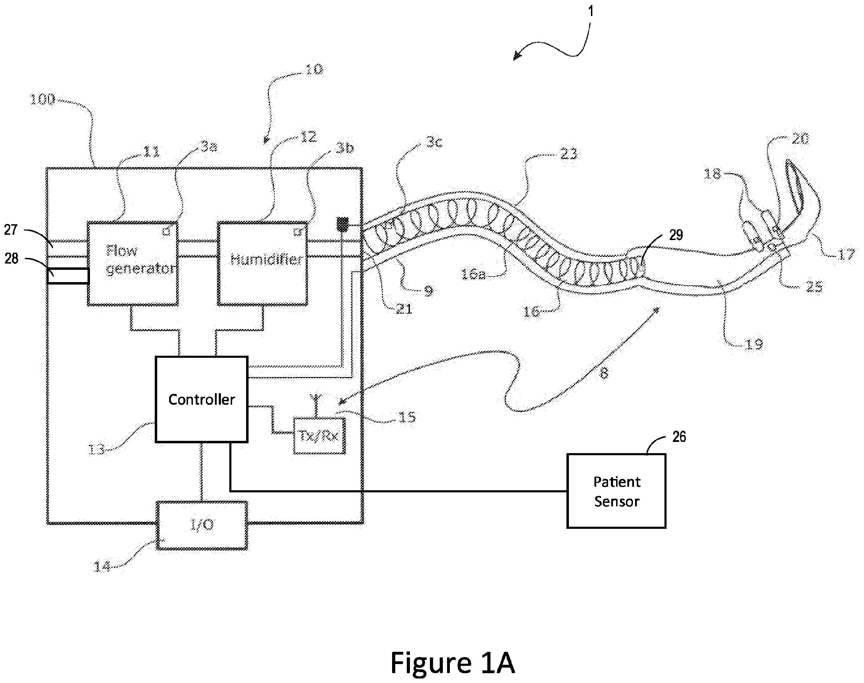

A respiratory therapy apparatus 10 is shown in Figure 1A. The respiratory therapy apparatus 10 can comprise a main housing 100 that contains a flow generator 11 in the form of a motor/impeller arrangement (for example, a blower), an optional humidifier 12, a controller 13, and a user interface 14 (comprising, for example, a display and input device(s) such as button(s), a touch screen, or the like). The controller 13 can be configured or programmed to control the operation of the respiratory therapy apparatus 10. For example, the controller 13 can control components of the respiratory therapy apparatus 10, including but not limited to: operating the flow generator 11 to create a flow of gas (gases flow) for delivery to a patient, operating the humidifier 12 (if present) to humidify and/or heat the generated gases flow, control a flow of oxygen into the flow generator blower, receiving user input from the user interface 14 for reconfiguration and/or user-defined operation of the respiratory therapy apparatus 10, and outputting information (for example on the display) to the user.

For these purposes, the controller 13 includes one or more computer processors 13a and associated non-transitory memory or storage medium storing processor executable instructions or code. The instructions, when executed by the one or more processors cause the respiratory therapy apparatus to effect the steps and processes described herein.

The user can be a patient, healthcare professional (for example a clinician), or anyone else interested in using the apparatus. As used herein, a "gases flow" can refer to any flow of gases that may be used in the breathing assistance or respiratory device, such as a flow of ambient air, a flow comprising substantially 100% oxygen, a flow comprising some combination of ambient air and oxygen, and/or the like.

A patient breathing conduit 16 is coupled at one end to a gases flow outlet 21 in the housing 100 of the respiratory therapy apparatus 10. The patient breathing conduit 16 is coupled at another end to a patient interface 17 such as a non-sealed nasal cannula with a manifold 19 and nasal prongs 18. Additionally, or alternatively, the patient breathing conduit 16 can be coupled to a face mask, a nasal mask, a nasal pillows mask, an endotracheal tube, a tracheostomy interface, and/or the like. The gases flow that is generated by the flow therapy apparatus 10 may be humidified, and delivered to the patient via the patient breathing conduit 16 through the patient interface 17. The patient breathing conduit 16 can have a heating element 16a to heat gases flow passing through to the patient. The heating element 16a can be under the control of the controller 13. In at least one configuration, the heating element 16a is a heater wire 16a. The patient breathing conduit 16 and/or patient interface 17 can be considered part of the respiratory therapy apparatus 10, or alternatively peripheral to it. The respiratory therapy system 1 may comprise the respiratory therapy apparatus 10, patient breathing conduit 16, and patient interface 17 together can form a respiratory therapy system.

The controller 13 can control the flow generator 11 to generate a gases flow of the desired flow rate. The controller 13 can also control a supplemental oxygen inlet to allow for delivery of supplemental oxygen. The humidifier 12 (if present) can humidify the gases flow and/or heat the gases flow to an appropriate level, and/or the like. The controller 13 can be configured to control the humidifier 12. The gases flow is directed out through the patient breathing conduit 16 and patient interface 17 to the patient. The controller 13 can also control a humidifier heating element 16b in the humidifier 12 and/or the heating element 16a in the patient conduit 16 to heat the gas to a desired temperature for a desired level of therapy and/or level of comfort for the patient. The controller 13 can be programmed with or can determine a suitable target temperature of the gases flow.

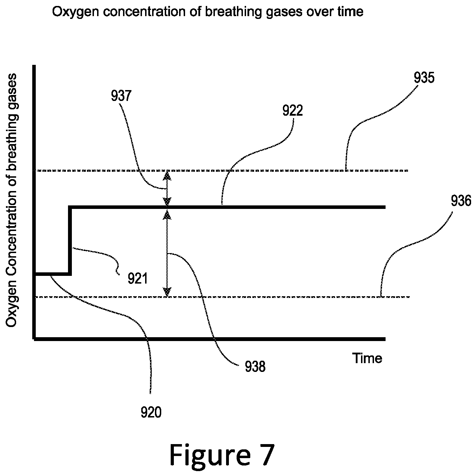

The oxygen inlet port 28 can include a valve through which a pressurized gas may enter the respiratory therapy apparatus 10. The valve can control a flow of oxygen into the respiratory therapy apparatus 10. The valve can be any type of valve, including a proportional valve or a binary valve. The source of oxygen can be an oxygen tank or a hospital oxygen supply. Medical grade oxygen is typically between 95% and 100% purity. Oxygen sources of lower purity can also be used. Examples of valve modules and filters are disclosed in U.S. Provisional Application No. 62/409,543, titled "Valve Modules and Filter", filed on October 18, 2016, and U.S. Provisional Application No. 62/488,841, titled "Valve Modules and Filter", filed on April 23, 2017. Valve modules and filters are discussed in further detail below with relation to Figures 7-15.

The flow therapy apparatus 10 can measure and control the oxygen content of the gas being delivered to the patient, and therefore the oxygen content of the gas inspired by the patient. In at least one implementation of high flow therapy, the high flow rate of gas delivered meets or exceeds the peak inspiratory demand of the patient. This means that the volume of gas delivered by the device to the patient during inspiration meets, or is in excess of, the volume of gas inspired by the patient during inspiration. High flow therapy can therefore help to prevent entrainment of ambient air when the patient breathes in, as well as flushing the patient's airways of expired gas. So long as the flow rate of delivered gas meets or exceeds peak inspiratory demand of the patient, entrainment of ambient air is prevented, and the gas delivered by the device is substantially the same as the gas the patient breathes in. As such, the oxygen concentration measured in the device, may be equivalent to a fraction of delivered oxygen (FdO2), and may be substantially the same as the oxygen concentration the patient is breathing, fraction of inspired oxygen (FiO2), and as such the terms may can be seen as equivalent.

Operation sensors 3a, 3b, 3c, such as flow, temperature, humidity, and/or pressure sensors can be placed in various locations in the flow therapy apparatus 10. Additional sensors (for example, sensors 20, 25) may be placed in various locations on the patient conduit 16 and/or patient interface 17 (for example, there may be a temperature sensor 29 at or near the end of the inspiratory tube). The sensors can be monitored by the controller 13. This can assist the controller 13 in operating the flow therapy apparatus 10 in a manner that provides suitable therapy. In some configurations, providing suitable therapy includes meeting a patient's peak inspiratory demand. The respiratory therapy apparatus 10 may have a transmitter, receiver and/or transceiver 15 to enable the controller 13 to receive signals 8 from the sensors and/or to control the various components of the flow therapy apparatus 10, including but not limited to the flow generator 11, humidifier 12, and heating element 16a, humidifier heating element 16b or accessories or peripherals associated with the flow therapy apparatus 10. Additionally, or alternatively, the transmitter, receiver and/or transceiver 15 may deliver data to a remote server or enable remote control of the respiratory therapy apparatus 10 or respiratory therapy system 1.

Oxygen may be measured by placing one or more gas composition sensors (such as an ultrasonic transducer system) after the oxygen and ambient air have been mixed. The measurement can be taken within the respiratory therapy apparatus 10, the patient breathing conduit 16, the patient interface 17, or at any other suitable location.

Oxygen concentration may also be measured by using flow rate sensors on at least two of the ambient air inlet conduit, the oxygen inlet conduit, and the patient breathing conduit to determine the flow rate of at least two gases. By determining the flow rate of both inlet gases or one inlet gas and one total flow rate, along with the assumed or measured oxygen concentrations of the inlet gases (about 20.9% for ambient air, about 100% for oxygen), the oxygen concentration of the final gas composition can be calculated. Alternatively, flow rate sensors can be placed at all three of the ambient air inlet conduit, the oxygen inlet conduit, and the final delivery conduit to allow for redundancy and testing that each sensor is working correctly by checking for consistency of readings. Other methods of measuring the oxygen concentration delivered by the flow therapy apparatus 10 can also be used.

The flow therapy apparatus 10 can include a patient sensor 26, such as a pulse oximeter or a patient monitoring system, to measure one or more physiological parameters of the patient, such as a patient's blood oxygen concentration (for example blood oxygen saturation (SpO2)), heart rate, respiratory rate, perfusion index, and provide a measure of signal quality. The sensor 26 can communicate with the controller 13 through a wired connection or by communication through a wireless transmitter on the sensor 26. The sensor 26 may be a disposable adhesive sensor designed to be connected to a patient's finger. The sensor 26 may be a non-disposable sensor. Sensors are available that are designed for different age groups and to be connected to different locations on the patient, which can be used with the flow therapy system 1. The pulse oximeter can be attached to the patient, typically at their finger, although other places such as an earlobe are also an option. The pulse oximeter can be connected to a processor in the respiratory therapy apparatus 10 and would constantly provide signals indicative of the patient's blood oxygen saturation. The patient sensor 26 can be a hot swappable device, which can be attached or interchanged during operation of the flow therapy apparatus 10. For example, the patient sensor 26 may connect to the flow therapy apparatus 10 using a USB interface or using wireless communication protocols (such as Bluetooth®). When the patient sensor 26 is disconnected during operation, the flow therapy apparatus 10 may continue to operate in its previous state of operation for a defined time period. After the defined time period, the flow therapy apparatus 10 may trigger an alarm, transition from automatic mode to manual mode, and/or exit control mode (e.g., automatic mode or manual mode) entirely. The patient sensor 26 may be a bedside monitoring system or other patient monitoring system that communicates with the flow therapy apparatus 10 through a physical or wireless interface.

The flow therapy apparatus 10 may comprise or be in the form of a high flow therapy apparatus. As used herein, "high flow" therapy refers to administration of gas to the airways of a patient at a relatively high flow rate that meets or exceeds the peak inspiratory demand of the patient. The flow rates used to achieve "high flow" may be any of the flow rates listed below. For example, in some configurations, for an adult patient 'high flow therapy' may refer to the delivery of gases to a patient at a flow rate of greater than or equal to about 10 litres per minute (10 LPM), such as between about 10 LPM and about 100 LPM, or between about 15 LPM and about 95 LPM, or between about 20 LPM and about 90 LPM, or between 25LPM and 75 LPM, or between about 25 LPM and about 85 LPM, or between about 30 LPM and about 80 LPM, or between about 35 LPM and about 75 LPM, or between about 40 LPM and about 70 LPM, or between about 45 LPM and about 65 LPM, or between about 50 LPM and about 60 LPM. In some configurations, for a neonatal, infant, or child patient 'high flow therapy' may refer to the delivery of gases to a patient at a flow rate of greater than 1 LPM, such as between about 1 LPM and about 25 LPM, or between about 2 LPM and about 25 LPM, or between about 2 LPM and about 5 LPM, or between about 5 LPM and about 25 LPM, or between about 5 LPM and about 10 LPM, or between about 10 LPM and about 25 LPM, or between about 10 LPM and about 20 LPM, or between about 10 LPM and 15 LPM, or between about 20 LPM and 25 LPM. A high flow therapy apparatus with an adult patient, a neonatal, infant, or child patient, may deliver gases to the patient at a flow rate of between about 1 LPM and about 100 LPM, or at a flow rate in any of the sub-ranges outlined above. The flow therapy apparatus 10 can deliver any concentration of oxygen (e.g., FdO2), up to 100%, at any flowrate between about 1 LPM and about 100 LPM. In some configurations, any of the flowrates can be in combination with oxygen concentrations (FdO2s) of about 20%-30%, 21%-30%, 21%-40%, 30%-40%, 40%-50%, 50%-60%, 60%-70%, 70%-80%, 80%-90%, and 90%-100%. In some combinations, the flow rate can be between about 25 LPM and 75 LPM in combination with an oxygen concentration (FdO2) of about 20%-30%, 21%-30%, 21%-40%, 30%-40%, 40%-50%, 50%-60%, 60%-70%, 70%-80%, 80%-90%, and 90%-100%. In some configurations, the flow therapy apparatus 10 may include safety thresholds when operating in manual mode that prevent a patient from delivering to much oxygen to the patient.

High flow therapy may be administered to the nares of a patient and/or orally, or via a tracheostomy interface. High flow therapy may deliver gases to a patient at a flow rate at or exceeding the intended patient's peak inspiratory flow requirements. The high flow therapy may generate a flushing effect in the nasopharynx such that the anatomical dead space of the upper airways is flushed by the high incoming gases flow. This can create a reservoir of fresh gas available for each and every breath, while minimizing re-breathing of nitrogen and carbon dioxide. Meeting inspiratory demand and flushing the airways is additionally important when trying to control the patient's FdO2. High flow therapy can be delivered with a non-sealing patient interface such as, for example, a nasal cannula. The patient interface 17 may be configured to deliver breathing gases to the nares of a patient at a flow rate exceeding the intended patient's peak inspiratory flow requirements.

The term "non-sealing patient interface" as used herein can refer to an interface providing a pneumatic link between an airway of a patient and a gases flow source (such as from flow generator 11) that does not completely occlude the airway of the patient. A non-sealed pneumatic link can comprise an occlusion of less than about 95% of the airway of the patient. The non-sealed pneumatic link can comprise an occlusion of less than about 90% of the airway of the patient. The non-sealed pneumatic link can comprise an occlusion of between about 40% and about 80% of the airway of the patient. The airway can include one or more of a nare or mouth of the patient. For a nasal cannula the airway is through the nares.

The flow generator 11 can be or comprises a blower module. The blower module may comprise at least one blower 11 configured to generate said flow of gases.

The flow generator 11 can include an ambient air inlet port 27 to entrain ambient room air into the blower. The flow therapy apparatus 10 may also include an oxygen inlet port 28 leading to a valve through which a pressurized gas may enter the flow generator 11. The valve can control a flow of oxygen into the flow generator 11. The valve can be any type of valve, including a proportional valve or a binary valve.

The blower 11 can operate at a motor speed of greater than about 1,000 RPM and less than about 30,000 RPM, greater than about 2,000 RPM and less than about 21,000 RPM, or between any of the foregoing values. Operation of the blower 11 can mix the gases entering the blower 11 through the inlet ports (for example ambient air inlet port 27 and/or an oxygen inlet port 28). Using the blower 11 as the mixer can decrease the pressure drop that would otherwise occur in a system with a separate mixer, such as a static mixer comprising baffles, because mixing requires energy.

The respiratory assistance apparatus may further comprises a gas composition sensor. The gas composition sensor may be the sensor described below (for example the ultrasonic transducer configuration).

The gas composition sensor may be connected to the controller.

The gas composition sensor may be located at any point in the flow path.

The gas composition sensor may be an oxygen concentration sensor configured to measure the oxygen concentration of the breathing gas.

The controller may be configured to receive an oxygen concentration signal indicative of the oxygen concentration of the breathing gas from the oxygen concentration sensor.

The gas composition sensor may be configured to provide a gas composition sensor output to the controller. Optionally, the gas composition sensor output may be a signal indicative of the gas composition (for example oxygen concentration) of the breathing gas.

The gas composition sensor may be in electronic communication with the controller.

The controller may be configured to control the oxygen concentration of the breathing gases based on the oxygen concentration signal from the oxygen concentration sensor.

The respiratory assistance apparatus may further comprises blood oxygen concentration sensor.

The blood oxygen concentration sensor may provide a signal to the controller.

The blood oxygen concentration sensor may be a pulse oximeter, or an arterial oxygen sensor.

The blood oxygen concentration sensor may be in electronic communication with the controller.

The controller 13 may comprise one or more processors. The processors may be configured with computer readable instructions.

The controller 13 may comprise at least one memory element. The memory element may be configured to store said computer readable instructions.

The memory element may be non-transitory.

The respiratory assistance apparatus may comprise at least one display module, configured to display an alarm output.

In some embodiments information relating to the estimated future value may be displayed. For example, the estimated minimum future value of a patient's blood oxygen and/or the estimated minimum future value of a patient's blood oxygen concentration, and/or the estimated future value.)

The respiratory assistance apparatus may comprise at least one audible module configured to emit an audible alarm.

The display module may comprise at least one display (for example a liquid crystal display (LCD), or an light emitting diode (LED) display, although it will be appreciated any display technology may be used)

The display module may be configured to receive inputs to the system (for example as a touch screen)

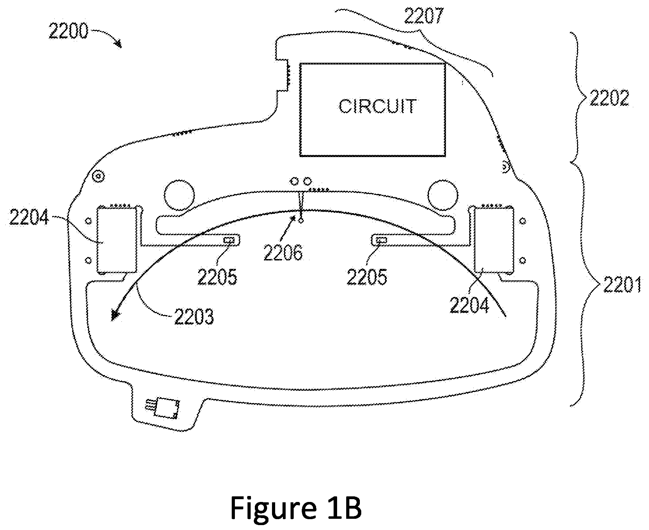

With additional reference to Figure 1B, a sensing circuit board 2200 is shown that can be implemented in the flow therapy apparatus 10. The sensing circuit board 2200 can be positioned in a sensor chamber such that the sensing circuit board 2200 is at least partially immersed in the flow of gases. The flow of gases may exit the blower 11 through a conduit and enter a flow path in the sensor chamber. At least some of the sensors on the sensing circuit board 2200 can be positioned within the flow of gases to measure gas properties within the flow. After passing through the flow path in the sensor chamber, the gases can exit to the humidifier 12 described above.

The sensing circuit board 2200 can be a printed sensing circuit board (PCB). Alternatively, the circuit on the board 2200 can be built with electrical wires connecting the electronic components instead of being printed on a circuit board. At least a portion of the sensing circuit board 2200 can be mounted outside of a flow of gases. The flow of gases can be generated by the flow generator 11 described above. The sensing circuit board 2200 can comprise ultrasonic transducers 2204. The sensing circuit board 2200 can comprise one or more of thermistors 2205. The thermistors 2205 can be configured to measure a temperature of the gases flow. The sensing circuit board 2200 can comprise a thermistor flow rate sensor 2206. The sensing circuit board 2200 can comprise other types of sensors, such as humidity sensors including humidity only sensors to be used with a separate temperature sensor and combined humidity and temperature sensors, sensors for measuring barometric pressure, sensors for measuring differential pressure, and/or sensors for measuring gauge pressure. The thermistor flow rate sensor 2206 can comprise hot wire anemometer, such as a platinum wire, and/or a thermistor, such as a negative temperature coefficient (NTC) or positive temperature coefficient (PTC) thermistor. Other non-limiting examples of the heated temperature sensing element include glass or epoxy-encapsulated or non-encapsulated thermistors. The thermistor flow rate sensor 2206 can be configured to measure flow rate of the gases by being supplied with a constant power, or be maintained at a constant sensor temperature or a constant temperature difference between the sensor and the flow of gases.

The sensing circuit board 2200 can comprise a first portion 2201 and a second portion 2202. The first portion 2201 can be positioned to be within the flow path of the gases, whereas the second portion 2202 can be positioned to be outside the flow path of the gases. The direction of the flow of gases is indicated in Figure 1B by the arrow 2203. The direction of the flow of gases can be a straight line, or curved in shown in Figure1B.

Positioning the one or more of thermistors 2205 and/or the thermistor flow rate sensor 2206 downstream of the combined blower and mixer can take into account heat supplied to the gases flow from the blower. Also, immersing the temperature-based flow rate sensors in the flow path can increase the accuracy of measurements because the sensors being immersed in the flow can more likely to be subject to the same conditions, such as temperature, as the gases flow and therefore provide a better representation of the gases characteristics.

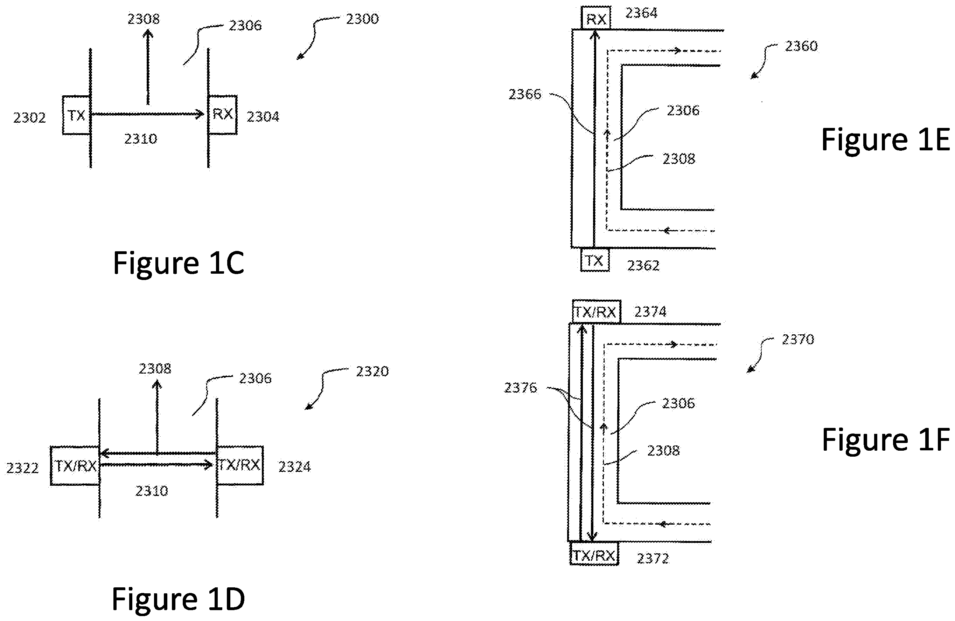

The sensing circuit board 2200 can comprise ultrasonic transducers, transceivers, or sensors of the sensing circuit board to measure gases properties of the gases flow, such as gas composition or concentration of one or more gases within the gases stream. Any suitable transducer, transceiver, or sensor may be mounted to the sensing circuit board 2200 as will be appreciated. In this configuration, the gas composition sensor is an ultrasonic transducer that employs ultrasonic or acoustic waves for determining gas concentrations. Various sensor configurations are described below with respect to Figures 1C-1F.

The ultrasonic transducer may determine the relative gas concentrations of two or more gases in the gases flow. The ultrasonic transducer may be configured to measure the oxygen fraction in the bulk gases stream flow, which consists of atmospheric air augmented with supplemental oxygen, which is essentially a binary gas mixture of nitrogen (N2) and oxygen (O2). It will also be appreciated that the ultrasonic transducer may be configured to measure the gas concentrations of other augmentation gases that have blended with atmospheric air in the gases stream, including nitrogen (N2) and carbon dioxide (CO2). The ultrasonic sensors can determine the gas concentration of gases in the gases flow at a relatively high frequency. For example, the ultrasonic sensors can output a measured FdO2 value at a maximum sample rate of the sensors or at a lower frequency than the maximum sample rate, such as between about 1 Hz and 200 Hz, about 1 Hz and 100 Hz, about 1 Hz and 50 Hz, and about 1 Hz and 25 Hz.

In some configurations, sensing circuit board 2200 includes a pair of ultrasonic transducers that are provided on opposite sides of the sensing circuit board. Various alternative configurations of the ultrasonic transducers can be used for sensing the characteristics of the gases stream by the transmission and reception of ultrasonic beams or pulses.

The distance between the ultrasonic transducers 2204 on opposite ends of the sensing circuit board 2200 can affect measurement resolution. An increased distance between each of the ultrasonic transducers 2204 can reduce the proportional or fractional error, since in general a measured length will have a certain amount of error, and if the length is increased, the proportion of error generated during measurement is less than for a shorter length. Thus, the overall uncertainty of the measurement decreases. An increased distance can also increase measurement resolution and accuracy, since it allows for a longer time period for acoustic signals between the ultrasonic transducers 2204. However, an increased distance can lead to a weaker signal.

The ultrasonic transducers 2204 can be positioned such that the space between the ultrasonic transducers 2204 at least partially coincides with the flow path. In some configurations, the ultrasonic transducers are positioned on opposing ends of the sensing circuit board. Because the whole face of the flow path is exposed to the acoustic path, the sound waves propagate through all of the gases in the flow path. Averaging of the waves can occur across the entire flow path rather than a section of the flow path. Averaging over a longer distance reduces error and reduces the dependence of air-oxygen mixing. The ultrasonic transducers can be configured to measure the gases characteristics from any angle relative to the flow path.

Positioning sensors in the flow path or module, instead of outside the flow path or module, allows the transducers 2204 to both operate within a smaller temperature range relative to one another, or both substantially at one temperature (namely, the temperature of the gas flow). Having them at a substantially homogenous temperature increases accuracy as the transducers are sensitive to temperature. Further, positioning sensors along the flow path allows for measurements and calculations that account for the influence of the gas velocity so that the effect of gas velocity can be removed from the sensor measurement.

Referring to Figures 1C-1F, various configurations of the ultrasonic transducers will be described for the gas composition sensing system for sensing the speed of sound through the gases stream by the transmission and reception of ultrasonic beams or pulses. Like reference numerals, represent like components.

Referring to Figure 1C, the transducer configuration 2300 provides an arrangement in which there is a pair of transducers 2302, 2304 opposing each from opposite sides of the sensing passage 2306, with the air flow path direction indicated generally by 2308. In this configuration, each of the transducers 2302, 2304 is driven as either a dedicated transmitter or receiver, such that ultrasonic pulses 2310 are transmitted uni-directionally across the gases flow path from the transmitter to the receiver transducer. As shown, the transducer pair is aligned (i.e. not-displaced upstream or downstream from each other) relative to the air flow path direction 2308 and is configured to transmit cross-flow pulses that are substantially perpendicular to the gases flow path direction.

Referring to Figure 1D, an alternative transducer configuration 2320 is illustrated in which a pair of transducers 2322, 2324 is provided opposing each other on opposite sides of the sensing passage, but wherein each transducer may operate as both a transmitter and receiver, i.e. is an ultrasonic transmitter-receiver or transceiver. In this configuration, bi-directional ultrasonic pulses 2326 may be sent between the transducer pair 2322, 2324. For example, pulses may be sent back and forth alternately between the transducers or in any other sequence or pattern. Again, the transducer pair is aligned relative to the gases flow path direction and are configured to transmit cross-flow pulses that are substantially perpendicular to the gases flow path direction.

Referring to Figure 1E, an alternative transducer configuration 2360 is illustrated in which there is a pair of transducers 2362, 2364 opposing each other from opposite ends of the sensing passage 2306, with the gases flow path direction or axis indicated generally by 2308. In this configuration 2360, each of the transducers 2362, 2364 is driven as either a dedicated transmitter or receiver, such that along-flow ultrasonic pulses 2366 are transmitted uni-directionally in a beam path between the transmitter and receiver that is substantially aligned or parallel with the gases flow path axis 2308 in the sensing passage 2306. In the embodiment shown, the transmitter is upstream of the receiver, but it will be appreciated that the opposite arrangement could be employed. With this configuration, a flow rate sensor is provided in the sensing passage to provide a flow rate signal indicative of the flow rate of the gases stream in the sensing passage. It will be appreciated that the speed of sound in the sensing passage can be derived or determined in a similar manner to that previously described with the previous embodiments, and that the flow rate signal is utilized in the signal processing to remove or compensate for the gases flow rate in the calculated speed of sound signal.

Referring to Figure 1F, an alternative transducer configuration 2370 is illustrated in which a pair of transducers 2372, 2374 is provided opposing each other from opposite ends of the sensing passage like in Figure 1E, but wherein each transducer may operate as both a transmitter and receiver, i.e. is an ultrasonic transmitter-receiver or transceiver. In this configuration, bi-directional along-flow ultrasonic pulses 2376 may be sent between the transducer pair 2372, 2374. For example, pulses may be sent back and forth alternately between the transducers or in any other sequence or pattern. Again, the transducer pair are aligned with the gases flow path axis 2308 and are configured to transmit cross-flow pulses in a beam path or paths that are substantially aligned or parallel to the gases flow path axis 2308 in the sensing passage 2306. With this configuration, a separate flow rate sensor need not necessarily be provided, as the flow rate component of the speed of sound signal can be directly derived or determined from processing of the transmitted and received acoustic pulses.

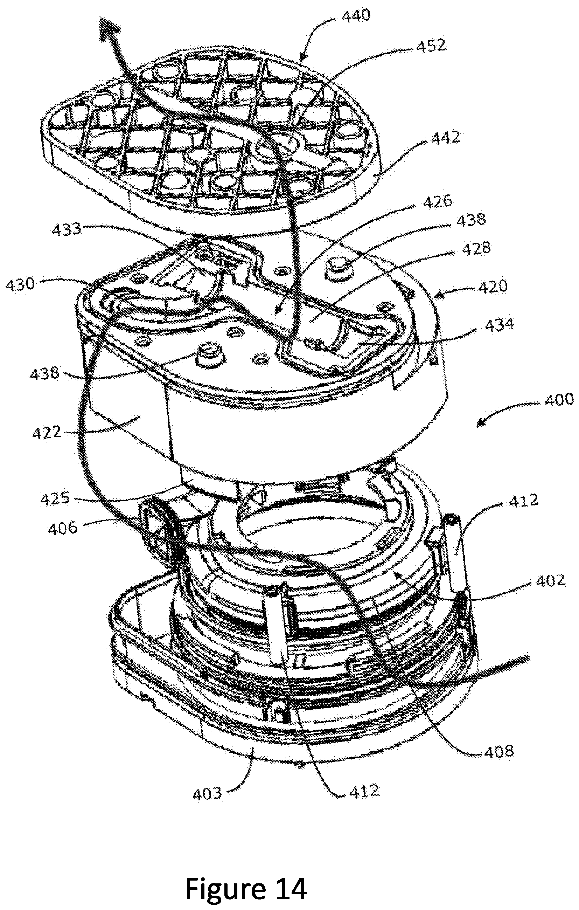

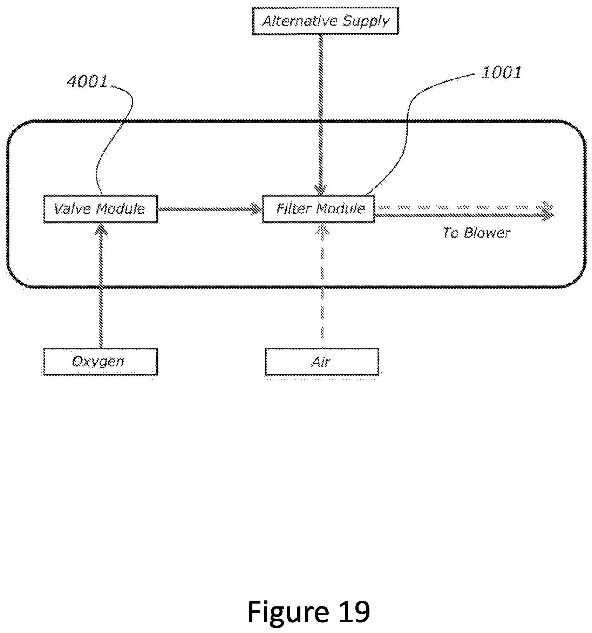

Some examples of flow therapy apparatuses are disclosed in International Application No. PCT/NZ2016/050193, titled "Flow Path Sensing for Flow Therapy Apparatus", filed on December 2, 2016, and International Application No. PCT/IB2016/053761, titled "Breathing Assistance Apparatus", filed on June 24, 2016. Examples of configurations of flow therapy apparatuses that can be used with aspects of the present disclosure are discussed in further detail below with relation to Figures 14-19.

With reference again to Figure 1A, the controller 13 can be programmed with or configured to execute a closed loop control system for controlling the operation of the flow therapy apparatus 10. The closed loop control system can be configured to ensure the patient's blood oxygen concentration (for example SpO2) reaches a target level and consistently remains at or near this level.

An oxygen control system is described in International Application No. PCT/NZ2018/050137, titled "Closed Loop Oxygen Control", filed on October 5, 2018.

The controller 13 may be configured to control the blood oxygen concentration of a patient to a target blood oxygen concentration (or a target blood oxygen concentration range.) The controller 13 may be configured to receive a signal indicative of the blood oxygen concentration of the patient, and calculate a target oxygen concentration of the breathing gases to control the blood oxygen concentration of a patient to a target blood oxygen concentration (or a target blood oxygen concentration range).

The target oxygen concentration of the breathing gases may be controlled to be within a target oxygen concentration range of the breathing gases (described in more detail below).

The controller 13 may be configured to control the oxygen concentration of the breathing gases to a target oxygen concentration of the breathing gases. The controller 13 may be configured to receive a signal indicative of the oxygen concentration of the breathing gases, and control the oxygen concentration of the breathing gases to the target oxygen concentration of the breathing gases.

Oxygen concentration of the breathing gases may be for example the measured oxygen concentration of the breathing gases, and/or the delivered oxygen concentration of the breathing gases, and/or the provided oxygen concentration of the breathing gases.

The controller 13 can receive input(s) from a user that can be used by the controller 13 to execute the closed loop control system. The input(s) may comprise a target blood oxygen concentration of a patient. The target blood oxygen concentration may be a target SpO2 value. The target blood oxygen concentration may be a single value or a range of values. The value(s) could be pre-set, chosen by a user, or determined based on the type of patient. The type of patient could refer to current affliction, and/or information about the patient such as age, weight, height, gender, and other patient characteristics. Similarly, the target SpO2 could be two values, each selected in any way described above. The two values would represent a range of acceptable values for patient's target blood oxygen concentration (described in more detail below). The controller can target a value within said range. The target blood oxygen concentration could be the middle value of the range, or any other value within the range, which could be pre-set or selected by a user. Alternatively, the range could be automatically set based on the target blood oxygen concentration. The controller can be configured to have one or more set responses when the patient's blood oxygen concentration moves outside of the range. The responses may include alarming, changing to manual control of FdO2, changing the FdO2 to a specific value, and/or other responses. The controller can have one or more ranges, where one or more different responses occur as it moves outside of each range.

The graphical user interface of the flow therapy apparatus 10 may be configured to prompt the user to input a patient type, and the target blood oxygen concentration (or for example the target blood oxygen concentration range) would be determined based on what the user selects. Additionally, the user interface may include a custom option, where the user can define the limits of the target oxygen concentration range of the breathing gases.

Generally, the patient's blood oxygen concentration (for example SpO2) would be controlled between about 80% and about 100%, or about 80% and about 90%, or about 88% and about 92%, or about 90% and about 99%, or about 92% and about 96%. The SpO2 could be controlled between any two suitable values from any two of the aforementioned ranges. The target SpO2 could be between about 80% and about 100%, or between about 80% and about 90%, or between about 88% and about 92%, or between about 90% and about 99%, or between about 92% and about 96%, or about 94%, or 94% or about 90%, or 90%, or about 85%, or 85%. The target SpO2 could be any value between any two suitable values from any two of the aforementioned ranges. The target SpO2 can correspond to the middle of a defined SpO2 range.

The input(s) received by the controller 13 may additionally or alternatively comprise a target oxygen concentration range. The target oxygen concentration range may be a target FdO2, or FiO2.

The target oxygen concentration range may be provided via a user interface 14.

As discussed above, the oxygen concentration of the breathing gases (for example FdO2) can be controlled within a target oxygen concentration range of the breathing gases. The target oxygen range may be an allowable range where the FdO2 is controlled within. As discussed previously, the measured in the apparatus (for example FdO2) would be substantially the same as the oxygen concentration the patient is breathing (FiO2) so long as the flow rate meets or exceeds the peak inspiratory demand of the patient, and as such the terms may can be seen as equivalent. Each of the limits of the target oxygen concentration range could be pre-set, selected by a user, or determined based on the type of patient. The type of patient could refer to current affliction, and/or information about the patient such as age, weight, height, gender, and/or other patient characteristic. Alternatively, a single value for FdO2 could be selected. The target oxygen concentration range can be determined at least partially based on this value. For example, the target oxygen concentration range could be a set amount above and below the selected FdO2. The selected FdO2 could be used as the starting point for the controller 13. The respiratory therapy apparatus 10 could have one or more responses if the controller 13 tries to move the FdO2 outside of the target oxygen concentration range. These responses could include alarming, preventing the FdO2 moving outside of the range, switching to manual control of FdO2, and/or switching to a specific FdO2. The respiratory therapy apparatus 10 could have one or more target oxygen concentration ranges where one or more different responses occur as it reaches the limit of each target oxygen concentration range.

The target oxygen concentration range of the breathing gases may comprise an upper target oxygen concentration.

The target oxygen concentration range of the breathing gases may comprise a lower target oxygen concentration.

It will be appreciated in certain circumstances no upper target oxygen concentration or lower target oxygen concentration may be provided.

Oxygen concentration of the breathing gases (for example FdO2) can be controlled between about 21% and about 100%, or about 21% and about 90%, or about 21% and about 80%, or about 21% and about 70%, or about 21% and about 60%, or about 21% and about 50%, or about 25% and about 45%. The FdO2 could be controlled between any two suitable values from any two ranges described. The FdO2 target could be between any two suitable values from any two ranges described. If the range is based on the single value, the upper and lower limits could be decided by adding/subtracting a fixed amount from the selected value. The amount added or subtracted could be about 1%, or about 5%, or 10 %, or about 15%, or about 20%, or about 30%, or about 50%, or about 100%. The amount added/subtracted could change relative to the selected value. For example, the upper limit could be 20% higher than the selected value, so a selected value of 50% FdO2 would have an upper limit of 60% for the range of control. The percentage used for the range could be about 1%, or about 5%, or 10 %, or about 15%, or about 20%, or about 30%, or about 50%, or about 100%. The method for calculating the lower limit and the upper would not necessarily need to be the same. If a single value is used, the value could be between about 21% and about 100%, or about 25% and about 90%, or about 25% and about 80%, or about 25% and about 70%, or about 25% and about 60%, or about 25% and about 50%, or about 25% and about 45%.

The graphical user interface 14 (GUI) can be configured to display the range of values between which FdO2 and/or SpO2 are being controlled. The range (for example the patient target blood oxygen range, or the target oxygen concentration range of the breathing gases) could be displayed by having the two limits set apart from each other on the GUI, with an indicator appearing within the respective range to graphically represent the position of the current value with respect to the limits of the range.

The GUI can display graphs of recent FdO2 and/or SpO2 data. The GUI can display the level of each parameter on the same or different graphs over a defined period of time, such as one or more hours. The length of time over which data is displayed could match the length of the time for which data is currently available.

FdO2 data displayed can be at least one of the target FdO2 or the measured FdO2. The SpO2 data can include a line indicating target SpO2. Additionally, or alternatively, SpO2 and/or FdO2 data can include one or more lines or shaded areas indicating their respective control limits.

The graphs can be displayed on the default display. Alternatively, the graphs can be hidden with only current data values being shown. The graph can become available through interaction with the GUI, such as by selecting to view a graph for a defined parameter.

The closed loop control system may utilize two control loops. The first control loop may be implemented by the blood oxygen concentration (for example SpO2) controller. The blood oxygen concentration controller can determine a target oxygen concentration (for example FdO2) based in part on the target blood oxygen concentration and/or the measured blood oxygen concentration. As discussed above, the target blood oxygen concentration value can be a single value or a range of acceptable values. The value(s) could be pre-set, chosen by a user, or determined automatically based on client characteristics.

The target blood oxygen concentration may be controlled within a target blood oxygen concentration range.

The target blood oxygen concentration range may comprise an upper target blood oxygen concentration and/or a lower target blood oxygen concentration.

The controller 13 may be configured to control the target blood oxygen concentration to be within the upper target blood oxygen concentration of the target blood oxygen concentration range and the lower target blood oxygen concentration of the target blood oxygen concentration range.

Generally, target blood oxygen concentration values are received or determined before or at the beginning of a therapy session, though target blood oxygen concentration values may be received at any time during the therapy session.

During a therapy session, the blood oxygen concentration controller can also receive as inputs: measured oxygen concentration reading(s) from a gases composition sensor, and measured blood oxygen concentration reading(s) and a signal quality reading(s) from the patient sensor. In some configurations, the blood oxygen concentration controller can receive target oxygen concentration as an input. In such a case, the output of the blood oxygen concentration controller may be provided directly back to the blood oxygen concentration controller as the input. Based at least in part on the inputs, the blood oxygen concentration controller can output a target oxygen concentration to the second control loop.

The second control loop may be implemented by a breathing gases oxygen concentration controller. The oxygen concentration controller may be configured to control the oxygen concentration from an initial oxygen concentration to a target oxygen concentration.

The blood oxygen concentration controller may be a separate controller to controller 13, or may be included as part of controller 13.

The oxygen concentration controller may be a separate controller to controller 13, or may be included as part of controller 13.

The initial oxygen concentration may be a measured oxygen concentration. In some embodiments the initial oxygen concentration will be the result of previous control of the oxygen concentration. In some embodiments for example at start-up of the system or when no supplemental oxygen source is connected, in this case the initial oxygen concentration may be substantially the same as that of ambient air.

The target oxygen concentration may be the target oxygen concentration (for example FdO2) provided by the blood oxygen concentration controller.

The oxygen concentration controller can receive inputs of measured oxygen concentration of the breathing gases and target oxygen concentration of the breathing gases. The breathing gases oxygen concentration controller can then output an oxygen inlet valve control signal to control the operation of the oxygen valve based on a difference between the measured oxygen concentration and target oxygen concentration values. The oxygen concentration controller may receive the target oxygen concentration value that is output from the first control loop when the respiratory therapy apparatus is operating in automatic mode. The breathing gases oxygen concentration controller may also receive additional parameters such as flow rate values, gas properties, and/or measured oxygen concentration. The gas properties may include the temperature of the gas at the O2 inlet and/or the oxygen content of the supply source. The gases supply source connected to the oxygen inlet valve may be an enriched oxygen gasflow where the oxygen content of the supply source may be less than pure oxygen (i.e., 100%). For example, the oxygen supply source may be an oxygen enriched gas flow having an oxygen content of less than 100% and greater than 21%.

From at least some of the inputs, the breathing gases oxygen concentration controller can determine an oxygen flow rate that would be required to achieve the target oxygen concentration. The oxygen concentration controller can use the flow rate input in order to alter the valve control signal. If the flow rate changes, the oxygen concentration controller can automatically calculate a new required oxygen flow rate required to maintain the target oxygen concentration at the new flow rate without having to wait for feedback from the gas concentration sensor, such as the measured oxygen concentration value. The oxygen concentration controller can then output the altered valve control signal to control the valve based on the new flow rate. In some configurations, the control signal of the oxygen concentration controller may set the current of the oxygen valve in order to control operation of the oxygen valve. Additionally, or alternatively, the oxygen concentration controller could detect changes to the measured oxygen concentration and alter the position of the valve accordingly. During manual mode, the second control loop can operate independently without receiving the target oxygen concentration from the first control loop. Rather, the target oxygen concentration can be received from user input or a default value.

During the therapy session, the blood oxygen concentration and oxygen concentration controllers can continue to automatically control the operation of the flow therapy apparatus until the therapy session ends or an event triggers a change from the automatic mode to manual mode.

The breathing gases oxygen concentration controller may be configured to be control oxygen concentration in the breathing gases to within a target oxygen concentration range. The target oxygen concentration range may comprise an upper target oxygen concentration, and/or a lower target oxygen concentration.

The respiratory assistance apparatus may further comprise at least one valving arrangement (as described in more detail below).

The valving arrangement may be controllable by the controller to vary the amount of supplemental oxygen provided from an oxygen source to the breathing gas.

The valving arrangement may be in fluid communication with the blower,

The valving arrangement may be controllable to adjust the amount of oxygen introduced into the gases flow.

The valving arrangement may comprise one or more actuators.

The controller may be configured to vary the amount of supplemental oxygen provided from an oxygen source to the breathing gas to control the oxygen concentration of the breathing gases.

The respiratory therapy system 1 may comprise a predictive alarm system. In particular, the respiratory therapy apparatus 10 may comprise the predictive alarm system. The predictive alarm system may generate an alarm based on an estimated future value of the patient's blood oxygen concentration.

The predictive alarm system may be part of the controller 13 or part of a separate alarm module.

The controller 13 may be configured to estimate the effect of a change in oxygen concentration of the breathing gases on a patient's blood oxygen concentration.

The controller 13 may be configured to estimate the effect of past changes (for example a series of changes) in oxygen concentration of the breathing gases on a patient's blood oxygen concentration.

The controller 13 may be configured to estimate a maximum future value of a patient's blood oxygen concentration based on the effect of past changes (for example a series of changes) in oxygen concentration of the breathing gases, and the target oxygen concentration range of the breathing gases.

The controller 13 may also take into account the patient blood oxygen (i.e. as measured by a sensor).

In some embodiments the patient blood oxygen may be determined over a period of time.

The controller 13 may be configured to control the oxygen concentration of the breathing gases to the target oxygen concentration.

The change in oxygen concentration of the breathing gases (the change in provided oxygen concentration) may cause a corresponding change in patient blood oxygen concentration.

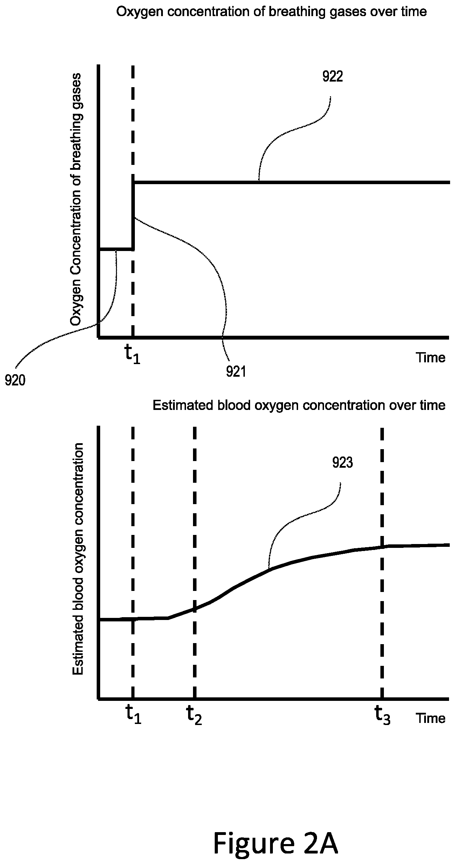

If the change is an increase in the oxygen concentration of the breathing gases (for example as shown in Figure 2A) then a corresponding increase in patient blood oxygen concentration should occur.

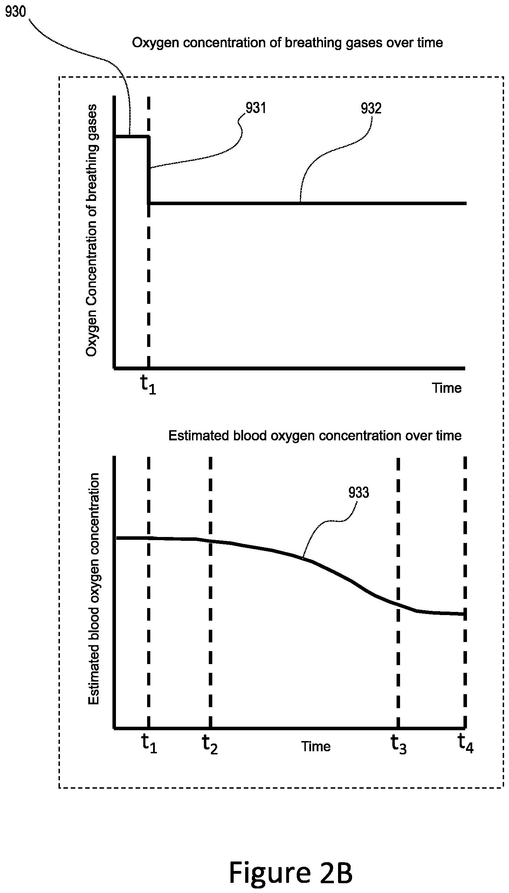

If the change is a decrease in the oxygen concentration of the breathing gases (for example as shown in Figure 2B then a corresponding decrease in patient blood oxygen concentration should occur.

Figure 2A shows an example increase (shown as a step change) in the oxygen concentration of the breathing gases over time (for example by the controller controlling to the target oxygen concentration of the breathing gases), and the corresponding effect this has on patient blood oxygen concentration over time.

At time t1 the controller 13 controls the oxygen concentration of the breathing gases from the initial oxygen concentration 920 through a step change 921 to a higher, target oxygen concentration 922. As shown in the blood oxygen concentration over time in Figure 2A, the effect the change in oxygen concentration 921 has on blood oxygen concentration is gradual over time, with the estimated blood oxygen concentration increasing over time from t1 to t3.

Figure 2B shows an example decrease (shown as a step change) in oxygen concentration of the breathing gases over time (for example by the controller controlling to the target oxygen concentration of the breathing gases), and the corresponding effect this has on patient blood oxygen concentration over time.

At time t1 the controller 13 controls the oxygen concentration of the breathing gases from the initial oxygen concentration 930 through a step change 931 to a lower, target oxygen concentration 932. As shown in the blood oxygen concentration over time the effect the change in oxygen concentration 931 has on blood oxygen concentration is gradual over time, with the estimated blood oxygen concentration 933 decreasing over time from t1 to t3. Time t4 represents a time where the change in oxygen concentration of the breathing gases has had its full effect on the estimated blood oxygen concentration 933.

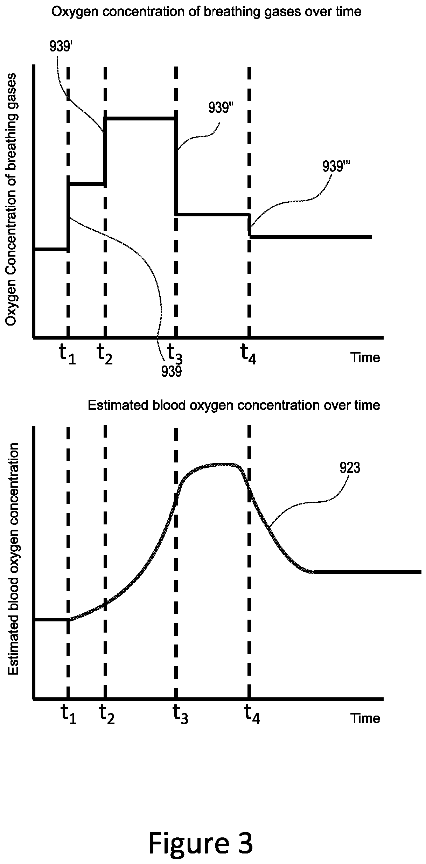

Figures 2A and 2B show a single step change in the oxygen concentration over time. It will be appreciated that the controller may execute a series of changes over a period of time (for example as shown in Figure 3).

Figure 3 shows a series of exemplary changes in the oxygen concentration of the breathing gases over time (for example the controller controlling to the target oxygen concentration of the breathing gases), and the corresponding effect this has on patient blood oxygen concentration over time.

At time t1 the controller 13 controls the oxygen concentration of the breathing gases through a step change 939 to a higher oxygen concentration of the breathing gases, followed by a further step change 939' at t2 to a higher oxygen concentration of the breathing gases, followed by a further step change 939" at t3 to a lower oxygen concentration of the breathing gases, followed by a further step change 939''' at t4 to a lower oxygen concentration of the breathing gases.

The controller 13 may be executing these changes to the oxygen concentration of the breathing gases based on control of the patient blood oxygen concentration (as described above)

The controller 13 may be executing these changes to reach a target oxygen concentration of the breathing gases.

Although figures 2A, 2B and 3 show changes to the oxygen concentration of the breathing gases as step changes, these changes may occur over a time period. In some embodiments control of the oxygen concentration of the breathing gases may be continuous.

During operation of the apparatus 10, the controller 13 may store the oxygen concentration of the breathing gases as stored oxygen concertation data.

The controller 13 may also store an associated timestamp. The controller 13 may store the oxygen concentration of the breathing gases and an associated timestamp as stored oxygen concentration data. For example the stored oxygen concentration data may comprise a one or a more oxygen concentrations of the breathing gases each oxygen concentration having an associated timestamp.

The timestamp may include information as to the time at when the oxygen concentration of the breathing gases was measured (for example by a sensor) or determined (for example calculated as a target.

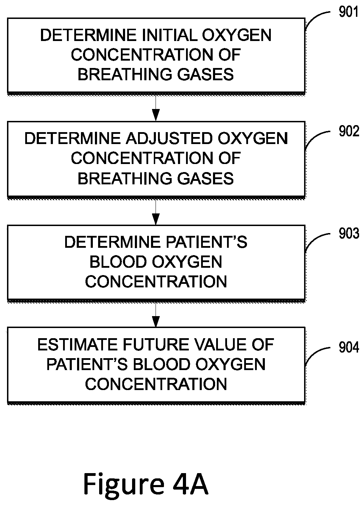

With reference to Figure 4A, the determination of the estimated future value of the patient's blood oxygen concentration is shown in more detail.

The determination of an estimated future value as shown in Figure 4A may be based on the effect of a single change in oxygen concentration of the breathing gases, or one or more changes in the oxygen concentration of the breathing gases over a period of time.

At block 901, the controller 13 determines the initial oxygen concentration of the breathing gases. As discussed above, if the flow therapy apparatus 10 has been operating, this initial oxygen concentration may be higher than that of ambient air. Alternatively, if the flow therapy apparatus 10 has not been operating, the oxygen concentration may be substantially the same as that of ambient air.

The initial oxygen concentration may be determined from a signal from the gases composition sensor (as described above). The controller 13 can monitor readings from the gases composition sensor to determine the initial oxygen concentration.

At block 902, the controller 13 determines the target oxygen concentration of the breathing gases. As discussed above, the target oxygen concentration of the breathing gases may be the target oxygen concentration (for example FdO2) provided by the blood oxygen concentration controller.

The target oxygen concentration may be determined from a signal from the gases composition sensor (as described above).

At block 903, the controller 13 determines the patient's blood oxygen concentration (for example the patient's SpO2). In some embodiments the patient's blood oxygen concentration may be determined over a predetermined time.

At block 904, the controller determines the estimated future value of the patient's blood oxygen concentration (described in more detail below).

The controller 13 may be configured to calculate an estimated future value of the patient's blood oxygen concentration based on a difference between the initial oxygen concentration of the breathing gases and the target oxygen concentration of the breathing gases.

Additionally, or alternatively, the controller 13 may be configured to calculate an estimated future value of the patient's blood oxygen concentration based on a measurement indicative of the patient's blood oxygen concentration.

In some embodiments, the estimated future value of the patient's blood oxygen concentration (BOC) may be based on the following equation:

$$Estimated\ future\ BOC = \ Current\ BOC + K(\Delta O_2\ Conc)$$

where:

Estimated future BOC is the estimated value of the patient's blood oxygen concentration,

Current BOC is the current patient blood oxygen concentration.

K is a factor or function which defines a relationship between the change in oxygen concentration of the supplied gas, and the effect on patient blood oxygen concentration.

$\Delta O_2$ Conc is the change in oxygen concentration of the breathing gases (for example the difference between the initial oxygen concentration of the breathing gases and the target oxygen concentration of the breathing gases).

The estimated future value of the patient's blood oxygen concentration may be further based on a time from when the controller controls the oxygen concentration of the breathing gases to the target oxygen concentration. Referring to the equation above, the factor K may be based on the time from when the change of oxygen concentration of the breathing gases occurred.

K may be a linear or non-linear function for example where an increase in supplied oxygen results in a corresponding increase in patient blood oxygen concentration.

The estimated future value of the patient's blood oxygen concentration may be based on an estimated oxygen efficiency for a patient. The estimated oxygen efficiency being an indication of a relationship between a change in oxygen concentration of the breathing gases and a change in patient blood oxygen concentration. The estimated oxygen efficiency for a patient may be included as part of the factor or function K as described above.

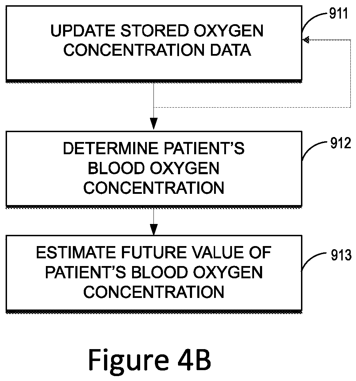

With reference to Figure 4B, another determination of the estimated future value of the patient's blood oxygen concentration is shown in more detail.

At block 911, the controller 13 may update stored breathing gases oxygen concentration data (as discussed in more detail above).

The controller 13 may store breathing gases oxygen concentration data from start-up of the apparatus, or from the initiation of therapy, or for a predetermined amount of time prior to the present time (for example for the last 15 minutes).

The controller 13 may update the stored breathing gases oxygen concentration data at regular time intervals, or irregular time intervals. In some embodiments, the controller may update the stored breathing gases oxygen concentration data in real time.

In some embodiments updating the stored breathing gases oxygen concentration data may comprise adding one or more further oxygen concentrations of the breathing gases and an associated timestamp (for example a newly measured oxygen concentration of the breathing gases along with when the measurement was made as the timestamp)

At block 912, the controller 13 determines the patient's blood oxygen concentration (for example the patient's SpO2).

At block 913, the controller determines the estimated future value of a patient's blood oxygen concentration (as described in more detail below).

In some embodiments, the estimated future value of the patient's blood oxygen concentration (BOC) may be based on the following equation:

$$Estimated\ future\ BOC = f(BOC,\ O_2\ Conc,\ C)$$

Where:

Estimated future BOC is the estimated value of the patient's blood oxygen concentration,

BOC is the blood oxygen concentration of the patient (for example the measured blood oxygen concentration), over the treatment period, or over a predetermined time,

$O_2$ Conc is the oxygen concentration of the breathing gases (for example the target oxygen concentration) over the treatment period, or over a predetermined time,

C may comprise constants and/or functions (for example and oxygen efficiency of a patient, and/or a haemoglobin saturation function).

This equation shows the estimated future BOC is a function of patient blood oxygen concentration, oxygen concentration of the breathing gases and constants and/or functions C.

In some embodiments the controller is configured to calculate an estimated future value of the patient's blood oxygen concentration based on the stored breathing gases oxygen concentration data, and a measurement indicative of the patient's blood oxygen concentration.

The oxygen concentrations of the breathing gases of the stored breathing gases oxygen concentration data may be weighted based on the associated timestamp.

The controller may determine a series of changes of the oxygen concentrations of the breathing gases are in the stored breathing gases oxygen concentration data.

The weighting based on the time stamp may be inversely proportional to the time since a change in oxygen concentration of the breathing gases has occurred.

That is, relatively older changes will be assigned a relatively lower weight in the calculation of the future value of blood oxygen concentration, as the effect of these changes may have already (at least partially) been realised on patient blood oxygen concentration, as compared to relatively more recent changes

In some embodiments, the estimated future value of the patient's blood oxygen concentration (BOC) may be based on a sum of changes of the oxygen concentration of the breathing gases.

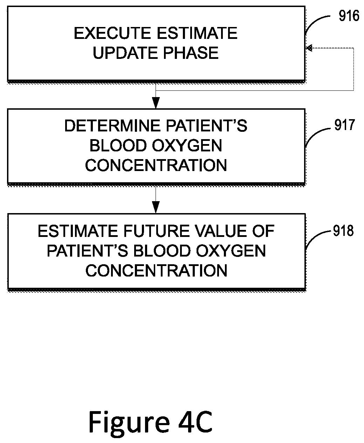

With reference to Figure 4C, another determination of the estimated future value of the patient's blood oxygen concentration is shown in more detail.

At block 916, the controller 13 executes an estimate update phase.

The estimate update phases may be configured to determine one or more variables of the apparatus 10 and update the sum of changes of the oxygen concentration of the breathing gases accordingly.

At block 917, the controller 13 determines a patient blood oxygen concentration.

At block 918, the controller 13 estimates a future value of the patient's blood oxygen concentration based on the sum of changes of the oxygen concentration (which has been updated in the estimate update phase at block 916).

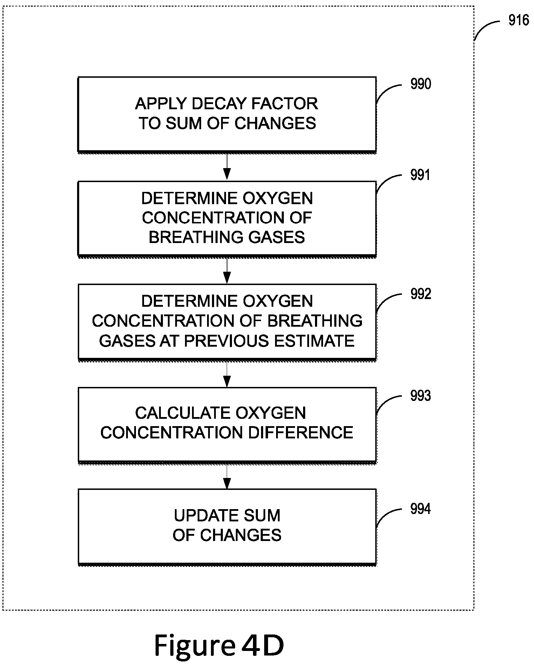

The estimate update phase 916 is shown in more detail in Figure 4D.

At block 990, a decay factor is applied to the current sum of changes in the oxygen concentration of the breathing gases.

The decay factor decays the sum of changes of the oxygen concentration of breathing gases at each estimate update phase to account for changes in oxygen concentration of the breathing gases which have had an effect on the patient blood oxygen concentration.

At block 991, the controller 13 determines the oxygen concentration of the breathing gases. As discussed above the oxygen concentration of the breathing gases may be a measured oxygen concentration (for example as measured by the gases composition sensor) or a target oxygen concentration (i.e. as set by the controller 13).

At block 992, the controller 13 determines the oxygen concentration of the breathing gases at the previous estimate update.

At block 993, the controller 13 determines a difference between the oxygen concentration of the breathing gases and the oxygen concentration of the breathing gases at the previous estimate update.

At block 994, the controller 13 updates the sum of changes of the oxygen concentration of breathing gases by adding the difference between the oxygen concentration of the breathing gases and the oxygen concentration of the breathing gases at the previous estimate update, and the decayed the sum of changes of the oxygen concentration of breathing gases.

The sum of changes of the oxygen concentration of the breathing gases may be indicative of the changes to the oxygen provided to the user which is yet to affect the patient's blood oxygen concentration.

The estimated future value of the patient's blood oxygen concentration may be based on the oxygen efficiency of a patient, and/or a haemoglobin saturation function.

The estimate update phase may be shown by the equation below.

$$\left(\sum \Delta O_2\ conc\right)^t = \propto \left(\sum \Delta O_2\ conc\right)^{t-1} + \left((O_2\ conc)^t - (O_2\ conc)^{t-1}\right)$$

Where:

$(\Sigma \Delta O_2\ conc)^t$ is the sum of changes of the oxygen concentration of the breathing gases at the current estimate update phase (at time t)

$\propto$ is the decay factor

$(\Sigma \Delta O_2\ conc)^{t-1}$ is the sum of changes of the oxygen concentration of the breathing gases at the previous estimate update phase (at time t-1)

$(O_2\ conc)^t$ is the oxygen concentration of the breathing gases at the current estimate update phase (at time t)

$(O_2\ conc)^{t-1}$ is the oxygen concentration of the breathing gases at the previous estimate update phase (at time t-1)

The controller 13 may be configured to execute the estimation update phase at regular time intervals, or irregular time intervals.

In some embodiments the controller 13 may be configured to execute the estimation update phase about every 0.5 second to about every 2 seconds, or every 1 second to around every 1.5 seconds, or every 0.5 seconds, or every 1 second, or every 1.5 second.

In some embodiments the decay factor may provide for an exponential decay.

The decay factor may be based on the time between estimation update phases.

In some embodiments, the decay factor may be chosen so any change in oxygen concentration of the breathing gases has its amplitude decayed to 36% of its original value in 45 seconds.

The stored oxygen concentration data may be transmitted by the respiratory apparatus device to one or more servers, or a patient monitoring unit, and/or a nurse monitoring station.

The controller 13 may be configured to compare the estimated future value of the patient's blood oxygen concentration with a blood oxygen concentration alarm range.

The controller 13 may generate an alarm output if the estimated future value of the patient's blood oxygen concentration is not within said blood oxygen concentration alarm range.

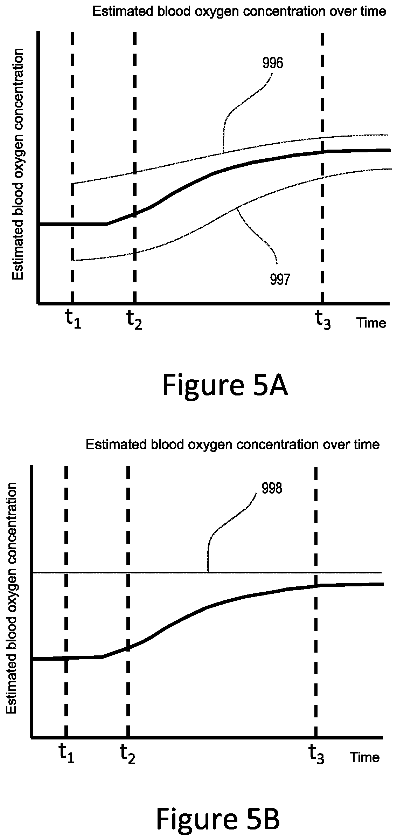

For example, in Figure 5A, the blood oxygen concentration alarm range may comprise an upper blood oxygen concentration alarm threshold 996, and a lower blood oxygen concentration alarm threshold 997.

The alarm output may be based on a difference between the estimated future value and the upper blood oxygen concentration alarm threshold 996 (or the lower blood oxygen concentration alarm threshold 997), and a measure of time.

The time taken for the alarm output to be generate may be inversely proportional to the difference. For example, if the difference is relatively small the alarm may take a longer time to activate, while if the difference is relatively larger the alarm may take a shorter time to activate.

In some embodiments, if the estimated blood oxygen concentration is close to the alarm threshold, the alarm will take a relatively long time to activate.

In some embodiments, if the estimated blood oxygen concentration is far from the alarm threshold, the alarm will take a relatively shorter time to activate.

The upper blood oxygen concentration alarm threshold 996 and/or lower blood oxygen concentration alarm threshold 997 may be based on the time from when the controller controls the oxygen concentration of the breathing gases to the target oxygen concentration. For example, the lower blood oxygen concentration alarm may increase over time.

The alarm threshold may be determined based on an amount of a change in target oxygen concentration (for example a difference between the target oxygen concentration and the initial oxygen concentration).

Additionally, or alternatively the alarm threshold may be determined or target oxygen concentration

Additionally, or alternatively the alarm threshold may be determined current oxygen concentration of gases.

The alarm threshold may be based on the target blood oxygen concentration.

As shown for example in Figure 5B, the controller 13 may be configured to generate an alarm output based on a comparison of the estimated future value of the patient's blood oxygen concentration with a blood oxygen concentration alarm threshold 998.

The controller 13 may be configured to generate said alarm output if the estimated future value of the patient's blood oxygen concentration is above or below the blood oxygen concentration threshold.

The alarm threshold may be based on the time from when the controller controls the oxygen concentration of the breathing gases to the target oxygen concentration.

The alarm output may be transmitted to one or more servers, or a patient monitoring unit, and/or a nurse monitoring station, in communication with the respiratory therapy apparatus.

The alarm threshold may be determined based on an amount of a change in target oxygen concentration (for example a difference between the target oxygen concentration and the initial oxygen concentration).

Additionally, or alternatively the alarm threshold may be determined or target oxygen concentration

Additionally, or alternatively the alarm threshold may be determined current oxygen concentration of gases.

The alarm range may be based on the target blood oxygen concentration.

The alarm output may be transmitted to one or more servers, or a patient monitoring unit, and/or a nurse monitoring station, in communication with the respiratory therapy apparatus.

The blood oxygen concentration alarm threshold may be based on the target blood oxygen concentration. For example, the blood oxygen concentration alarm threshold may be a percentage difference, or a predetermined amount from the target blood oxygen concentration.

The controller may also be configured to determine the effect of any further possible change to the oxygen concentration of the breathing gases within the target oxygen concentration range of the breathing gases on patient blood oxygen concentration.

For example, if the upper target oxygen concentration of the target oxygen concentration range is higher than the oxygen concentration of the breathing gases then the controller can determine the effect on patient blood oxygen concentration of an increase in oxygen concentration of the breathing gases to the upper target oxygen concentration.

The alarm output is described in more detail below.

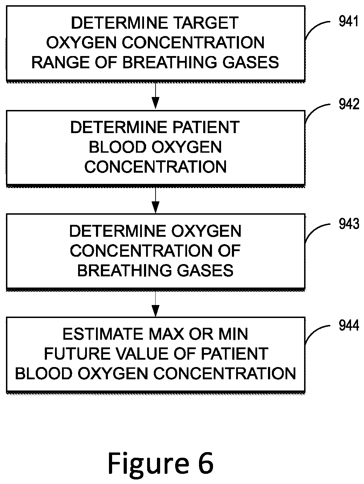

With reference to Figure 6, the determination of the maximum or minimum future value of the patient's blood oxygen concentration for a corresponding target oxygen range is shown in more detail.

The maximum or minimum future value may be after an effect of any change of oxygen concentration of the breathing gases has been realised on patient blood oxygen (for example when the patient's blood oxygen no longer changes, or reaches a steady state).

At block 941, the controller 13 determines the target oxygen concentration range of the breathing gases (described in more detail above). For example, the target oxygen concentration range may be provided as an input from a user or may be determined by the controller 13 or another controller of the apparatus 13.

At block 942, the controller 13 determines the patient blood oxygen concentration (for example, with a sensor such as a pulse oximeter).

At block 943, the controller 13 determines the oxygen concentration of the breathing gases. If the controller 13 is in the process of changing or controlling the oxygen concentration of the breathing gases, the oxygen concentration of the breathing gases may be a target oxygen concentration of the breathing gases.

At block 944, the controller 13 determines an estimated maximum or minimum future value of patient blood oxygen concentration. The estimated maximum or minimum future value of patient blood oxygen concentration may be based on the target oxygen concentration range of the breathing gases, the current oxygen concentration of the breathing gases, and the current patient blood oxygen concentration.

Figure 7 shows an example of the controller 13 controlling an oxygen concentration of the breathing gases from an initial oxygen concentration 920 to an target oxygen concentration 921. The target oxygen concentration 921 may be within the target oxygen concentration range. As discussed above, the target oxygen concentration range may comprise the upper target oxygen concentration 935 and/or the lower target oxygen concentration 936.

The controller may determine a difference between the upper target oxygen concentration 935 and the target oxygen concentration 921 (as shown by reference 937.)

The controller may determine a difference between the lower target oxygen concentration 936 and the target oxygen concentration 921 (as shown by reference 938.)

The controller 13 may be configured to calculate an estimated maximum or minimum future value of a patient's blood oxygen concentration for the target oxygen concentration range. That is, calculate an estimation of a maximum or minimum future value of a patient's blood oxygen concentration given a possible further change of provided oxygen concentration that the apparatus can provide while still being within the target oxygen concentration range.

This estimated maximum or minimum future value can be useful in providing information to a user as to the estimated effect that the apparatus can provide with respect to the patient's blood oxygen concentration within the target oxygen concentration range of the breathing gases input by the user. For example, if the estimated maximum or minimum future value is not acceptable to the user then the user can be notified and change the input parameters of the system, or modify the therapy.

The controller 13 may be configured to calculate an estimated maximum future value of a patient's blood oxygen concentration for the target oxygen concentration range. The estimated maximum future value of a patient's blood oxygen concentration for the target oxygen concentration range may be based on a difference between the target oxygen concentration and the upper target oxygen concentration, and the measurement indicative of a patient's blood oxygen concentration.

The estimated maximum or minimum future value of a patient's blood oxygen concentration may additionally be based on a measurement indicative of a patient's blood oxygen concentration.

The determinations of the estimated maximum or minimum future value of a patient's blood oxygen concentration may comprise aspects of the methods described above to determine an estimated future value of patient blood oxygen concentration.

The estimated maximum or minimum future value of a patient's blood oxygen concentration may be based on changes to the oxygen concentration of the breathing gases which have occurred in the past (for example the stored breathing gases oxygen concentration data), and potential changes which could occur in the future (for example changes which could occur in control of the oxygen concentration of the breathing gases within the target oxygen concentration range of the breathing gases).

In some embodiments, the maximum estimated future value of the patient's blood oxygen concentration (BOC) may be based on the following equation:

$$BOC_{max}^{est} = Current\ BOC + P(\text{upper target } O_2 \text{ conc} - \text{ current } O_2 \text{ conc})$$

where:

$BOC_{max}^{est}$ is the estimated maximum value of the patient's blood oxygen concentration for the corresponding target oxygen range.

Current BOC is the current patient blood oxygen concentration.

P is a factor or function which defines a relationship between the further potential increase in oxygen concentration of the supplied gas, and the effect on patient blood oxygen concentration.

Upper target $O_2$ conc is the upper target oxygen concentration (optionally of the target oxygen concentration range).

Current $O_2$ conc is the current oxygen concentration of the breathing gases.

The controller 13 may be configured to calculate an estimated minimum future value of a patient's blood oxygen concentration for the target oxygen concentration range. The estimated minimum future value of a patient's blood oxygen concentration may be based on a difference between the target oxygen concentration and the lower target oxygen concentration, and the measurement indicative of a patient's blood oxygen concentration.

In some embodiments, the maximum estimated future value of the patient's blood oxygen concentration may be based on the following equation:

$$BOC_{min}^{est} = Current\ BOC + Q(\text{lower target } O_2 \text{ conc} - \text{ current } O_2 \text{ conc})$$

where:

$BOC_{min}^{est}$ is the estimated minimum value of the patient's blood oxygen concentration for the corresponding target oxygen range.

Current BOC is the current patient blood oxygen concentration.

Q is a factor or function which defines a relationship between the further potential decrease in oxygen concentration of the supplied gas, and the effect on patient blood oxygen concentration.

Lower target $O^2$ conc is the lower target oxygen concentration, optionally of the target oxygen concentration range.

Current $O^2$ conc is the current oxygen concentration of the breathing gases.

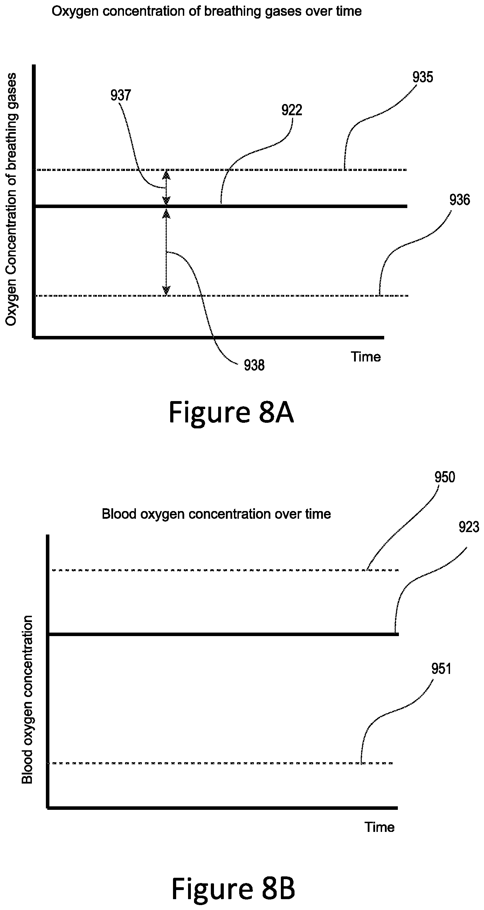

Figure 8A shows the target oxygen concentration range of the breathing gases defined by an upper target oxygen concentration 935 and a lower target oxygen concentration 936.

An example of the calculation of the estimated minimum or maximum future value of a patient's blood oxygen concentration is shown in Figures 8A and 8B. Figure 8A shows the oxygen concentration of the breathing gases 922 over time. In this example the controller 13 is controlling the oxygen concentration of the breathing gases 922 to constant value (i.e. there is no change).

The oxygen concentration of the breathing gases 922 corresponds with a blood oxygen concentration 932 of a patient over time. As the oxygen concentration of the breathing gases 922 is constant so it the blood oxygen concentration 932 of the patient.

As described above, the controller 13 may calculate a difference 937 between the upper target oxygen concentration 935 of the breathing gases and the oxygen concentration of the breathing gases 922 (for example, the current oxygen concentration of the breathing gases). This difference 937 may then be used, along with the measurement indicative of a patient blood oxygen concentration 923, to estimate a maximum future value of a patient's blood oxygen concentration 950.

As described above the controller 13 may calculate a difference 938 between the lower target oxygen concentration 936 of the breathing gases and the oxygen concentration of the breathing gases 922 (for example, the current oxygen concentration of the breathing gases). This difference 938 may then be used, along with the measurement indicative of a patient blood oxygen concentration 923, to estimate a minimum future value of a patient's blood oxygen concentration 951.

In some embodiments the estimated maximum or minimum future value of a patient's blood oxygen concentration may be based on an estimated future value of the patient's blood oxygen concentration (based on a recent change in oxygen concentration) for example, as described above. This allows the estimation of a maximum or a minimum future value of a patient's blood oxygen concentration to account for past or recent changes in oxygen concentration of the breathing gases (as described above).

In some embodiments, the estimated maximum or minimum future value of a patient's blood oxygen concentration may be based on the stored breathing gases oxygen concentration data (as described in more detail above).

In some embodiments, the estimated maximum or minimum future value of a patient's blood oxygen concentration may be based on the sum of changes of the oxygen concentration of the breathing gases (as described in more detail above).

The estimated maximum or minimum future value of a patient's blood oxygen concentration may additionally be based on a measurement indicative of a patient blood oxygen concentration and the estimated future value of the patient's blood oxygen concentration (as described above).

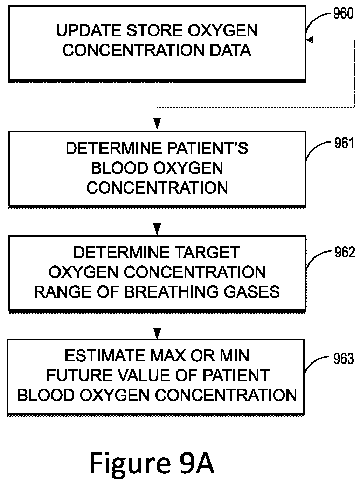

With reference to Figure 9A, the determination of the maximum or minimum future value of the patient's blood oxygen concentration, for a corresponding target oxygen concentration range of the breathing gases, is shown in more detail.

At block 960, the controller 13 may update the stored breathing gases oxygen concentration data (as discussed in more detail above).

The controller 13 may store breathing gases oxygen concentration data from start-up of the machine, or from the initiation of therapy, or for a predetermined amount of time from the present time (for example for the last 15 minutes).

The controller 13 may store breathing gases oxygen concentration data at regular time intervals, or irregular time intervals. In some embodiments, the controller may update the stored breathing gases oxygen concentration data in real time.

At block 961, the controller 13 determines the patient's blood oxygen concentration (for example the patient's SpO2).

At block 962, the controller 13 determines the target oxygen concentration range of the breathing gases (described in more detail above). For example, the target oxygen concentration range of the breathing gases may be provided as an input from a user or may be determined by the controller 13 or another controller of the apparatus 13.

At block 963, the controller 13 calculates an estimated maximum or minimum future value of a patient's blood oxygen concentration (as described elsewhere in the specification).

In some embodiments, the estimated maximum or minimum future value of the patient's blood oxygen concentration (BOC) may be based on the following equation:

$$Estimated\ maximum\ or\ minimum\ future\ BOC = f(BOC, \quad O_2\ Conc, \quad Target\ O_2\ Conc, C)$$

Where:

Estimated maximum or minimum future BOC is the estimated maximum or minimum future value of the patient's blood oxygen concentration,

BOC is the blood oxygen concentration of the patient, over the treatment period, or over a predetermined time,

$O_2$ Conc is the oxygen concentration of the breathing gases over the treatment period, or over a predetermined time,

Target $O_2$ Conc is the target oxygen concentration range of the breathing gases

C may comprise constants and/or functions (for example and oxygen efficiency of a patient, and/or a haemoglobin saturation function.)

This equation shows the estimated maximum or minimum future BOC is a function of patient blood oxygen concentration, oxygen concentration of the breathing gases, the target oxygen concentration range of the breathing gases, and constants and/or functions C.

In some embodiments the controller is configured to calculate the estimated maximum or minimum future value of the patient's blood oxygen concentration based on the stored breathing gases oxygen concentration data, the target oxygen concentration range of the breathing gases, and a measurement indicative of the patient's blood oxygen concentration.

The oxygen concentrations of the breathing gases of the stored breathing gases oxygen concentration data may be weighted based on the associated timestamp.

The controller may determine a series of changes of the oxygen concentrations of the breathing gases are in the stored breathing gases oxygen concentration data.

The weighting based on time stamp may be inversely proportional to the time since a change in oxygen concentration of the breathing gases has occurred.

That is, relatively older changes will be assigned a relatively lower weight in the calculation of the estimated maximum or minimum future blood oxygen concentration, as the effect of these changes may have already (at least partially) been realised on patient blood oxygen concentration, as compared to relatively more recent changes

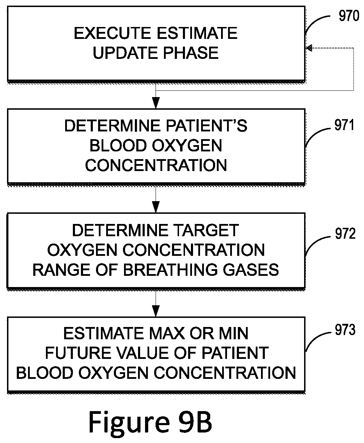

With reference to Figure 9B, another determination of the estimated maximum or minimum future value of the patient's blood oxygen concentration is shown in more detail.

At block 970, the controller 13 executes an estimate update phase (as discussed in more detail above and as shown in Figure 4C).

At block 971, the controller 13 determines a patient blood oxygen concentration.

At block 972, the controller 13 determines the target oxygen concentration range of the breathing gases.

At block 973, the controller 13 estimates a future value of the patient's blood oxygen concentration based on the sum of changes of the oxygen concentration (which has been updated in the estimate update phase at block 916).

The controller 13 may calculate a difference 937 between the upper target oxygen concentration 935 of the breathing gases and the oxygen concentration of the breathing gases 922 (for example, the current oxygen concentration of the breathing gases). This difference 937 may then be used, along with the measurement indicative of a patient blood oxygen concentration 923, and the sum of changes of the oxygen concentration of the breathing gases, to estimate a maximum future value of a patient's blood oxygen concentration 950.

The controller 13 may calculate a difference 938 between the lower target oxygen concentration 936 of the breathing gases and the oxygen concentration of the breathing gases 922 (for example, the current oxygen concentration of the breathing gases). This difference 938 may then be used, along with the measurement indicative of a patient blood oxygen concentration 923, and the sum of changes of the oxygen concentration of the breathing gases, to estimate a minimum future value of a patient's blood oxygen concentration 951.

The estimated maximum future value and/or the estimated minimum future value may be updated in real time. In this way the estimated maximum future value and/or the estimated minimum future value may be constantly updated during operation of the apparatus 10.

The estimated maximum future value and/or the estimated minimum future value may be based on an oxygen efficiency ratio.

In some embodiments the oxygen efficiency ratio (including the oxygen efficiency ratio as described above) may be input by a user.

In some embodiments the oxygen efficiency ratio may be calculated based on a measured blood oxygen concentration and a lower target oxygen concentration of the breathing gases (or an upper target oxygen concentration of the breathing gases).

In another embodiment, the flow therapy apparatus 10 can determine an oxygen efficiency associated with the patient.

The system can calculate an estimate of the patient's oxygen efficiency ($\xi$O2), along with other parameters. Generally, the oxygen efficiency can be calculated based on the patient's measured blood oxygen concentration (for example SpO2) and the measured oxygen concentration of the breathing gases (for example FdO2). In one configuration, the oxygen efficiency is determined based on the patient's measured SpO2 divided by the measured FdO2.

A patient in need of supplementary oxygen may have an oxygen efficiency that is less than that of a healthy individual. For example, in a healthy individual, a change in FdO2 could cause double the change in SpO2 as it would for a patient SpO2 with low oxygen efficiency. Having a measure of the patient's oxygen efficiency allows for a more efficient execution of a closed loop oxygen control system.

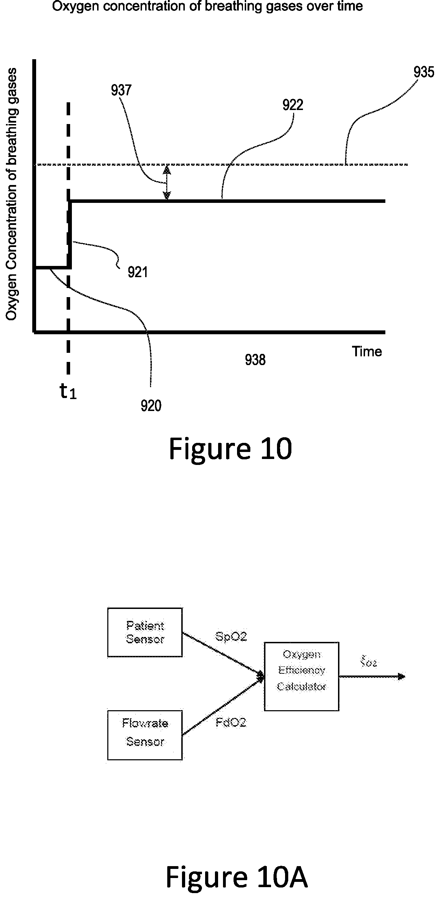

As illustrated in Figure 10A, the controller 13 can calculate an oxygen efficiency for the patient. The controller can receive the measured SpO2 value. The measured FdO2 value can be received from the gases composition sensors. An instantaneous oxygen efficiency can then be calculated based on the measured SpO2 the measured FdO2 values. The patient's overall oxygen efficiency can then be estimated by applying a running filter to the instantaneous oxygen efficiency data. Filtering the instantaneous oxygen efficiency data can reduce fluctuations in the estimate of the patient's overall oxygen efficiency. The controller may also prioritize more recent data. The instantaneous oxygen efficiency data can be weighted by the pulse oximeter's signal quality, such that measures of instantaneous oxygen efficiency that were made from data with low signal quality can have a reduced effect on the estimate of the patient's overall oxygen efficiency. The instantaneous oxygen efficiency data can also be weighted based on the size of recent changes to FdO2, such that measures of instantaneous oxygen efficiency that were made from data following a large change in FdO2 can have a reduced effect on the estimate of the patient's overall oxygen efficiency. This is because of a delay between when a change is made in the FdO2 and when there is a change in the measured SpO2. The controller can also take into account whether or not the patient is wearing the cannula when estimating the patient's oxygen efficiency. For example, the controller can disregard efficiency data from periods when the patient is not wearing the cannula.

The device can constantly monitor and update the estimate of the patient's overall oxygen efficiency. The patient's overall oxygen efficiency may be used by multiple parts of the closed loop control system, such as in the predictive model, tuning of the PID coefficients, and/or the step up of the feed forward phase. The patient's overall oxygen efficiency can be constantly updated as estimates of the patient's instantaneous oxygen efficiency changes. The controller can start with an initial estimate of the patient's oxygen efficiency based on the typical oxygen efficiency for a patient requiring supplemental oxygen. The overall oxygen efficiency can then be updated as data is received. A higher estimate of the oxygen efficiency can result in smaller changes in FdO2, which can reduce risk to the patient of receiving too much oxygen. A lower estimate can result in larger changes in FdO2, which can allow the controller to achieve the target SpO2 more quickly but may cause overshoot.

In some configurations, the flow therapy apparatus 10 can have an initial oxygen efficiency calculation phase in order to determine an oxygen efficiency of the patient. In some configurations, the oxygen efficiency is not updated after the initial oxygen efficiency calculation.

The controller may be configured to generate an alarm output.

The alarm output may comprise an alarm signal.

The alarm output may have any of the features as described above.

The alarm output may be provided in combination with the above described alarm output.

The controller 13 may be configured to compare the estimated maximum future value of a patient's blood oxygen concentration with the upper target blood oxygen concentration, and generate the alarm output if the estimated maximum future value of a patient's blood oxygen concentration is greater than the upper target blood oxygen concentration.

The controller 13 may be configured to compare the estimated maximum future value of a patient's blood oxygen concentration with the lower target blood oxygen concentration, and generate the alarm output if the estimated maximum future value of a patient's blood oxygen concentration is less than the lower target blood oxygen concentration.

An alarm parameter of the alarm output may be based on the magnitude of the difference between the estimated maximum future value of a patient's blood oxygen concentration and the upper target blood oxygen concentration.

The controller 13 may be configured to compare said estimated minimum future value of a patient's blood oxygen concentration with the lower target blood oxygen concentration, and generate an alarm output if the estimated minimum future value of a patient's blood oxygen concentration is less than the lower target blood oxygen concentration.

The controller 13 may be configured to compare said estimated minimum future value of a patient's blood oxygen concentration with the upper target blood oxygen concentration, and generate an alarm output if the estimated minimum future value of a patient's blood oxygen concentration is greater than the upper target blood oxygen concentration.

An alarm parameter of the alarm output may be based on the magnitude of the difference between the estimated minimum future value of a patient's blood oxygen concentration with the lower target blood oxygen concentration.

The alarm parameter may be the alarm duration, or the alarm intensity.

The alarm intensity may be a level of alarm, for example indicating the seriousness of the alarm.

The alarm output may be provided to a display.

The display may be configured to provide a user interface.

The alarm output may be configured to generate a sound, or provide an indication (for example a visual indication).

The alarm output may be transmitted to one or more servers, or a patient monitoring unit, and/or a nurse monitoring station, in communication with the respiratory therapy apparatus.

The alarm output may be based on a difference between the estimated maximum future value and the lower blood oxygen concentration alarm threshold 931(or a difference between the estimated minimum future value and the upper blood oxygen concentration alarm threshold 930), and a measure of time.

The time taken for the alarm output to generate may be inversely proportional to the difference. For example, if the difference is relatively small, the alarm may take a longer time to activate, while if the difference is relatively larger, the alarm may take a shorter time to activate.

In some embodiments, if the estimated blood oxygen concentration is close to the alarm threshold, the alarm will take a relatively long time to activate.

In some embodiments, if the estimated blood oxygen concentration is far from the alarm threshold, the alarm will take a relatively shorter time to activate.

An under oxygenation warning alarm output may be provided when the controller 13 predicts that the estimated maximum future value of a patient's blood oxygen concentration is more than a threshold amount below the target blood oxygen concentration.

The under oxygenation warning alarm output may be provided after a under oxygenation warning alarm output time has elapsed during which the estimated maximum future value of a patient's blood oxygen concentration is more than a threshold amount below the target blood oxygen concentration.

The under oxygenation warning alarm threshold amount may have a first threshold corresponding to an under oxygenation first level warning and a second threshold corresponding to an under oxygenation second level warning, where the first threshold is greater than the second threshold.

The under oxygenation first level warning may be indicative of a large difference between the estimated maximum future value of a patient's blood oxygen concentration and the target blood oxygen concentration.

The under oxygenation second level warning may be indicative of a smaller difference between the estimated maximum future value of a patient's blood oxygen concentration and the target blood oxygen concentration.

The under oxygenation first level warning may have a different alarm parameter to the under oxygen second level warning (for example a longer alarm time).

An over oxygenation warning alarm output may be provided when the controller 13 predicts estimated minimum future value of a patient's blood oxygen concentration is more than a threshold amount above the target blood oxygen concentration.

The over oxygenation warning alarm output may be provided after an over oxygenation warning alarm output time has elapsed during which the estimated minimum future value of a patient's blood oxygen concentration is more than a threshold amount above the target blood oxygen concentration.

The over oxygenation warning alarm threshold amount may have a first threshold corresponding to an over oxygen first level warning and a second threshold, and an over oxygenation second level warning, where the first threshold is greater than the second threshold.

The over oxygenation first level warning may be indicative of a large difference between the estimated minimum future value of a patient's blood oxygen concentration and the target blood oxygen concentration.

The over oxygenation second level warning may be indicative of a smaller difference between the estimated minimum future value of a patient's blood oxygen concentration and the target blood oxygen concentration.

The over oxygenation first level warning may have a different alarm parameter to the over oxygen second level warning (for example, a longer alarm time).

An alarm output may also be generated as per the equation below.

$$\Sigma_{Alarm} = \sum (BOC_{min} - Estimated\ Maximum\ or\ minimum\ future\ value - \beta)$$

Where

$\Sigma_{Alarm}$ is an alarm sum,
$BOC_{min}$ is a minimum BOC threshold, for example as a user input, or as a lower target blood oxygen concentration,
*Estimated Maximum or minimum future value* is an estimated max or min future value as described above
$\beta$ is a sensitivity factor.

The alarm output may be generated where the alarm sum is above a threshold.

Although the above description is related to an apparatus, it will be appreciated that the above could be implemented as a method.

Also disclosed is a flow therapy apparatus used for providing the method as described above.

The above mentioned embodiments may at least in part performed on an auxiliary device 2.

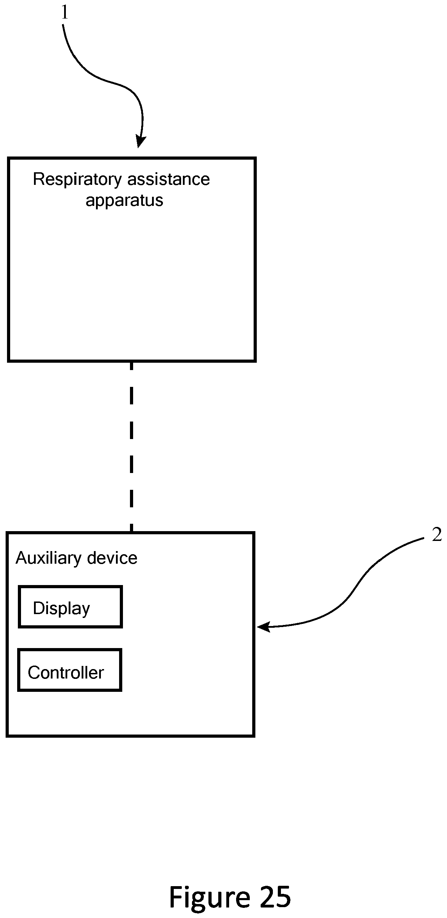

For example calculating estimated maximum future value, and/or the estimated minimum future value, and/or the estimated future value may be performed on the auxiliary device (for example as shown in Figure 25) as opposed to the controller of the respiratory assistance device.

As shown in Figure 25 the auxiliary device 2 may comprise a display and/or a controller. The controller for example may undertake any of the functions of the controller 13 of the respiratory assistance apparatus 1.

As shown in Figure 25 the respiratory assistance apparatus may provide information to the auxiliary device 2 to provide for the information needed to calculate the estimated future value of a patient's blood oxygen.

For example the respiratory assistance apparatus 1 may provide variables measured by the respiratory assistance apparatus 1 such as the current patient blood oxygen concentration, and the target oxygen concentration of the breathing gases, to the auxiliary device so that the auxiliary device 2 may perform the calculation.

The respiratory assistance apparatus 1 may also provide other information to be displayed on the auxiliary device 2 (for example patient blood oxygen concentration, and the target oxygen concentration of the breathing gases or other variables measured or calculated by the respiratory assistance apparatus 1).

In some embodiments, the respiratory apparatus 1 may communicate an alarm output (as described in more detail above) to the auxiliary device 2.

The auxiliary device 2 may output an alarm (for example on an audio and/or visual alarm via a display and/or speaker of the auxiliary device.)

In some embodiments, the auxiliary device 2 may determine an alarm output and provide the alarm output to the respiratory assistance apparatus 1. The respiratory apparatus 1 may then output the alarm as described above)

The auxiliary device 2 may be or comprise a server, or a patient monitoring unit, and/or a nurse monitoring station and/or a mobile device such as a tablet or mobile phone.

The auxiliary device 2 may communicate with the respiratory apparatus 1 via a wired connection or via a wireless connection (for example Bluetooth or NFC).

The auxiliary device 2 may be provided with an app or user interface to display information and/or alarms.

The auxiliary device 2 may be configured to vary at least one parameter of the respiratory assistance apparatus (for example a target oxygen concentration of the breathing gases).

## Motor and/or Sensor Module Configuration

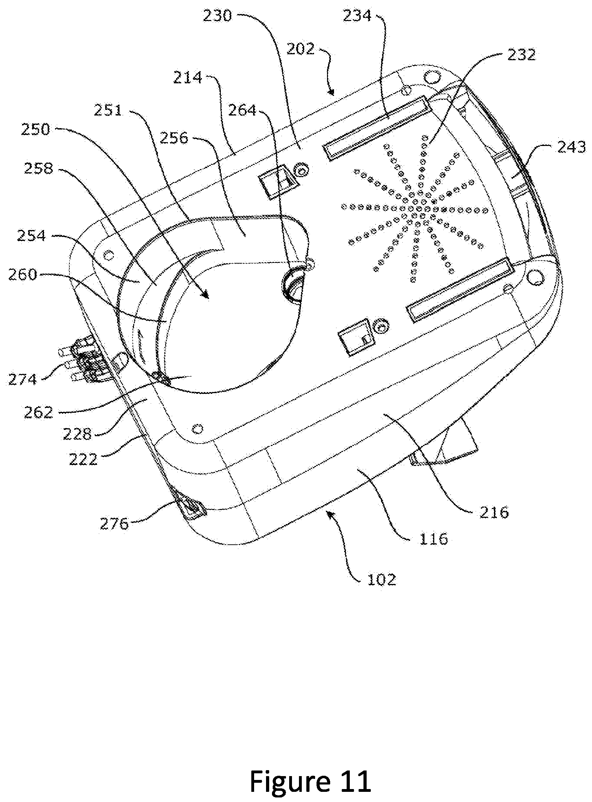

A configuration of a flow therapy apparatus 10 is illustrated in Figures 11 to 13. The flow therapy apparatus comprises a main housing 100. The main housing 100 has a main housing upper chassis 102 and a main housing lower chassis 202.

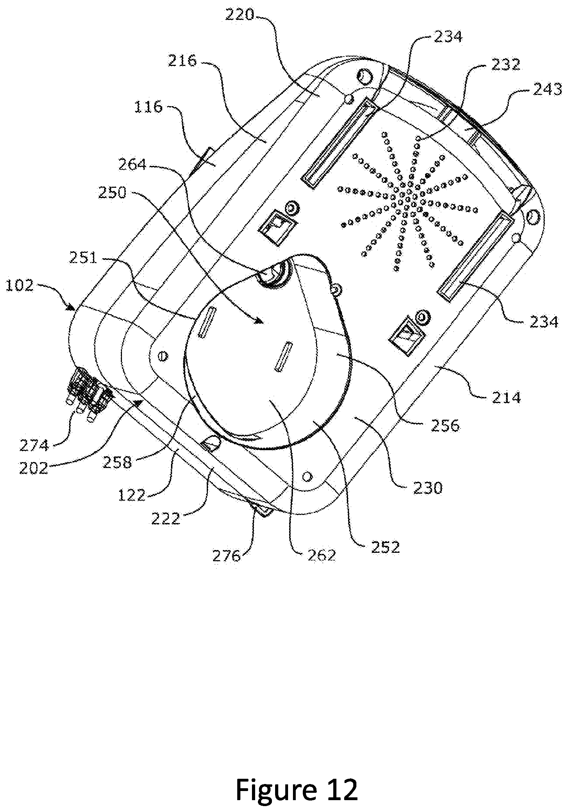

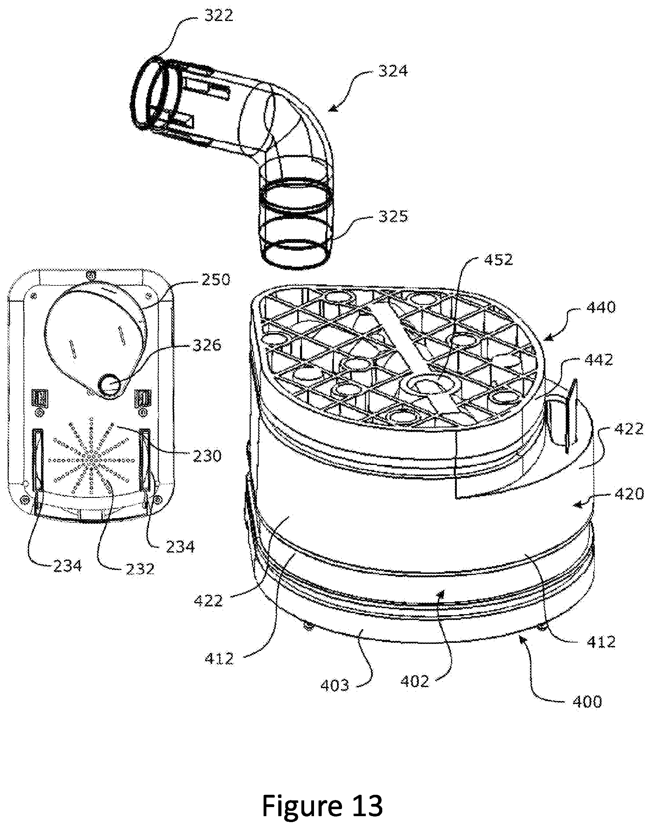

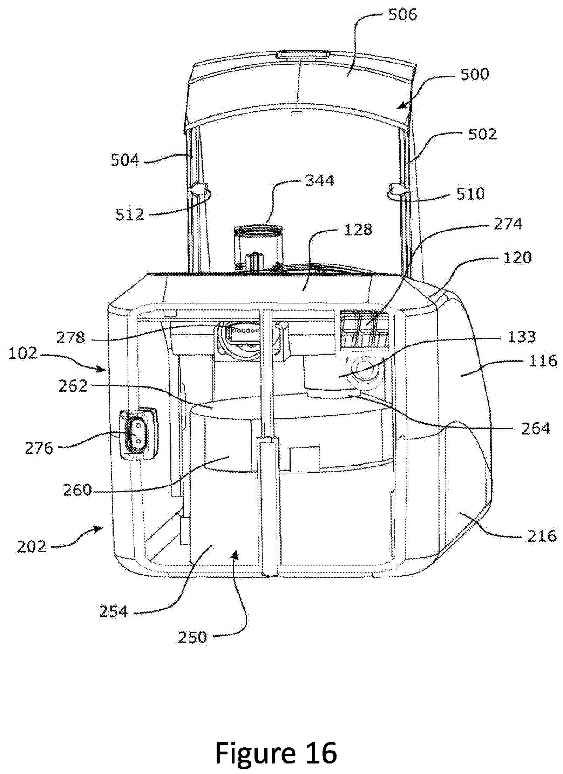

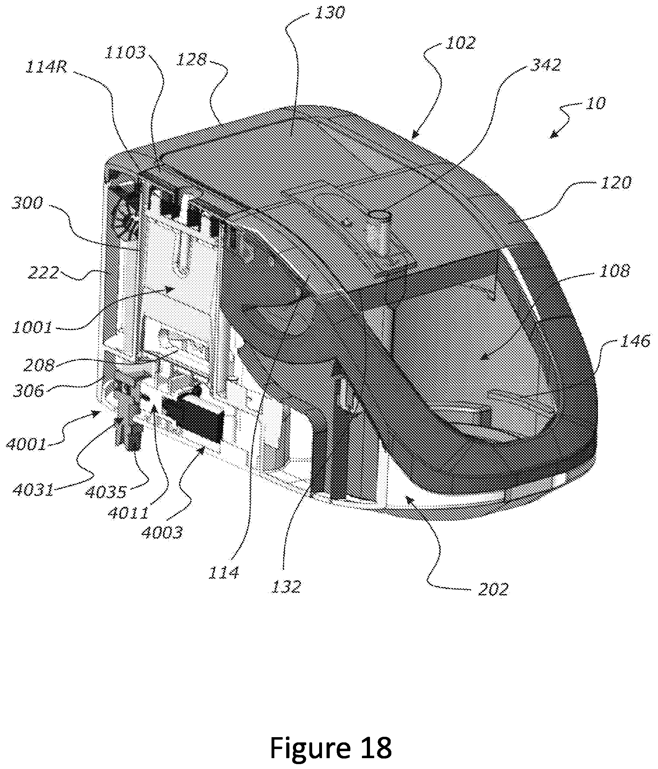

As shown in figures 11 and 12, the lower chassis 202 has a motor recess 250 for receipt of a removable or non-removable motor and/or sensor module 400 which is shown in figures 13 to 15 and will be described in further detail below. A recess opening 251 is provided in the bottom wall 230 adjacent a rear edge thereof, for receipt of a removable or non-removable motor/sensor module 400 which is shown in figures 16 and 18 and will be described in further detail below.

Figures 13 to 16 show the motor and/or sensor module or sub-assembly 400 in greater detail. As discussed above, the lower chassis 202 comprises a recess 250 for receipt of the motor and/or sensor module 400.

In the form shown in figures 13 to 16, the motor and/or sensor module 400 comprises a stacked arrangement of three main components; a base 403 of the sub-assembly 400 (on which is positioned the motor 402), an outlet gas flow path and sensing layer 420 positioned above the base 403, and a cover layer 440. The base 403, the sensing layer 420, and the cover layer 440 assemble together to form a sub-assembly housing that has a shape that is complementary to that of the recess 250 so that the sub-assembly 400 can be received in the recess 250. The base 403 is configured to close the recess opening 251 when the sub-assembly 400 is positioned in the recess 250. The sub-assembly 400 may be maintained in position in the recess in any suitable way such as with fasteners, clips, or a quick release arrangement for example, or fixed in a non-removable manner.

The sensing layer comprises a gas flow path with one or more sensors, the gas flow path arranged to deliver gas to the outlet port of the housing.

The motor 402 has a body 408 that defines an impeller chamber that contains an impeller. The motor 402 could be any suitable gas blower motor, and may for example be a motor and impeller assembly of the type described in published PCT specification WO2013/009193.

A gases outlet 406 is in fluid communication with a gases inlet of the outlet gas flow path and sensing layer 420, which is stacked on top of the motor. This layer 420 comprises a body 422 which comprises a plurality of mounting legs 425 that can be inserted into a plurality of mounting slots (not shown) of the base 403 to secure the body 422 to the base 403. In one configuration, the body 422 defines a gas flow path that couples the gases outlet 406 with the gases inlet of the gas flow path and sensing layer 420.

The body 422 defines a lower portion 426 of a sensing and gas flow path. The cover layer 440 has a body 442 that defines the upper portion 446 of the sensing and gas flow path, with the shape of the upper and lower portions 426, 446 corresponding substantially to each other.

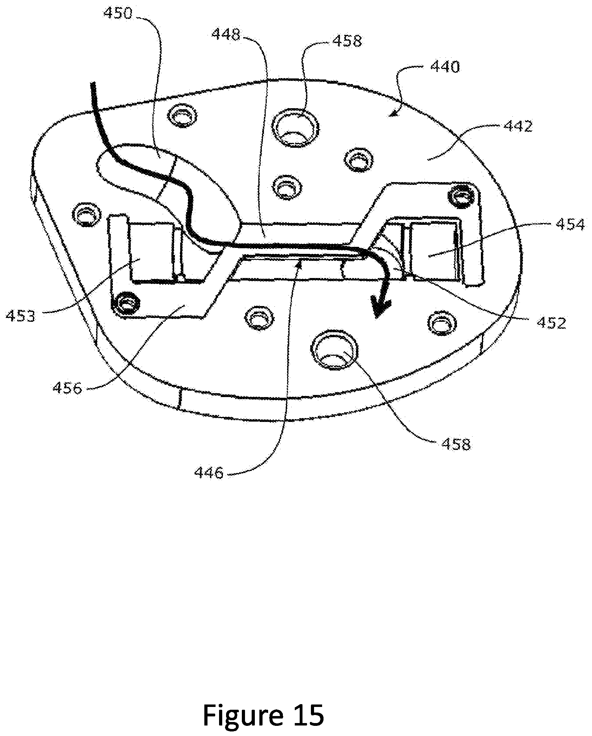

As shown in figures 14 and 15, the gas flow path comprises a linear elongate gas flow portion 428, 448. The inlet is in fluid communication with a tangential entrance portion 430, 450 of the gas flow path, which is located at or adjacent an entrance end of the linear elongate portion 428, 448 of the gas flow path. Recesses 433, 453 and 434, 454 may be provided at opposite ends of the linear elongate portion of the gas flow path.

A gas flow outlet port 452 extends vertically through the body 442 of the cover layer 440, and is located at or adjacent an opposite exit end of the linear elongate portion 428, 448 of the gas flow path. The gas outlet port 452 is in fluid communication with an upper portion of the motor recess 250, which in turn is in fluid communication with the gas flow passage. Again, due to the wall 252 and ceiling 262 configuration of the recess 250, if there is gas leakage from the motor/sensor module 400, that will be vented to atmosphere rather than entering the portion of the main housing 100 that contains the bulk of the electronics and control equipment. The recess 250 may comprise spacer(s), such as lugs that protrude downwardly from ceiling 262 as shown in figure 15, to maintain a suitable spacing for gas flow from the gas outlet port 452 and the ceiling of the recess 262.

It can be seen from figure 14 that that at least part of the gas flow path through and out of the motor and/or sensing module 400 has a tortuous or sinuous configuration. For example, the direction of gas flow travel through the elongate portions 428, 448 is generally opposite to the direction of gas flow travel from the gas outlet port 452 to the entrance of the gas flow passage through elbow 324.

As shown in figures 14 and 15, the cover layer 440 comprises a sensing printed circuit board (PCB) 456. The cover layer 440 may also comprise one or more temperature sensors such as thermistors that sit in the elongate portion 428, 448 of the gas flow path. One sensor will measure gas temperature and the other can act as a redundant temperature sensor. Alternatively, one of the thermistors could be used as a reference flow sensor (e.g. via use as a constant-temperature thermistor), and the measured temperatures could be used to determine the gas flow rate through the portion 428, 448 of the gas flow path. The one or more temperature sensors may be located on a portion of the sensing PCB 456 that faces the gas flow. The sensing PCB 456 may additionally comprise other sensors including but not limited to pressure sensors, humidity sensors and dew point sensors.

One or both of the electronics boards 272 will be in electrical communication or coupled with the sensors to process information received from the sensors and operate the apparatus 10 based on the information received from the sensors.

In an alternative configuration, the motor/impeller unit may be provided remotely from the apparatus 10. In that configuration, the module received in the recess 250 may only comprise a gas flow path and various sensors, to deliver gases to the fixed elbow 324 and thereby to the liquid chamber 300. In an alternative configuration, the module received in the recess 250 may only comprise the motor and a gas flow path, but no sensors.

In another alternative configuration the motor and/or sensor module 400 may not be removable from the recess 250, but instead may be permanently mounted therein. The benefits of the gas isolation from the electrical/electronics components would still be provided in that configuration.

The flow path is compact, and has reduced turns/sharp turns which reduces flow separation and reduces resistance to flow.

The arrangement of the motor and flow path provides another layer of isolation because of the wall arrangement.

Having a modular motor and/or sensor module enables the various parts of the module to be taken apart if needed for cleaning and/or servicing.

There are advantageously no leak paths in the motor and/or sensor module. While the motor and/or sensor module may be a potential leak point, a leak in that region would result in the oxygen venting to atmosphere or into the liquid chamber.

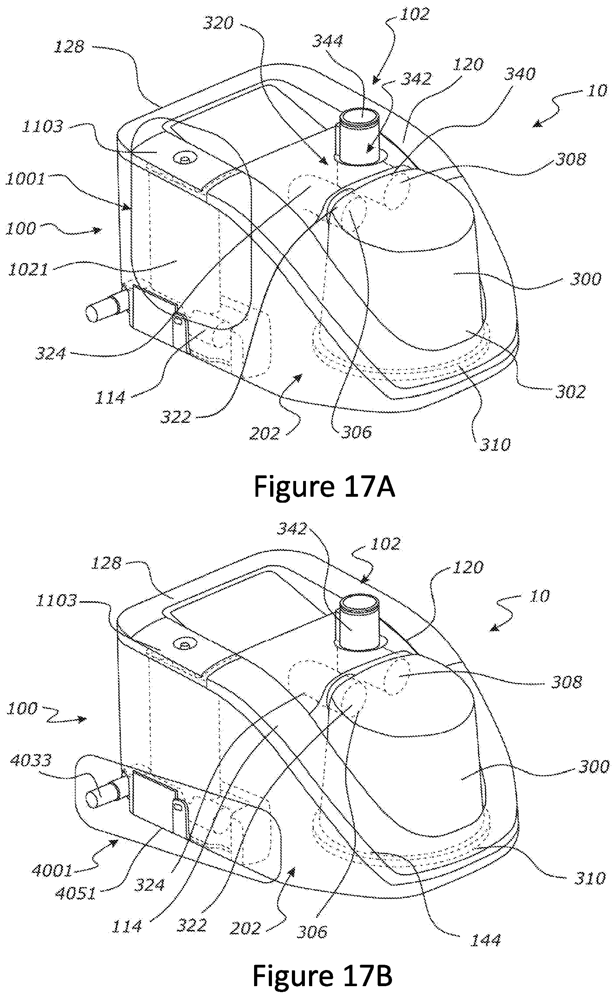

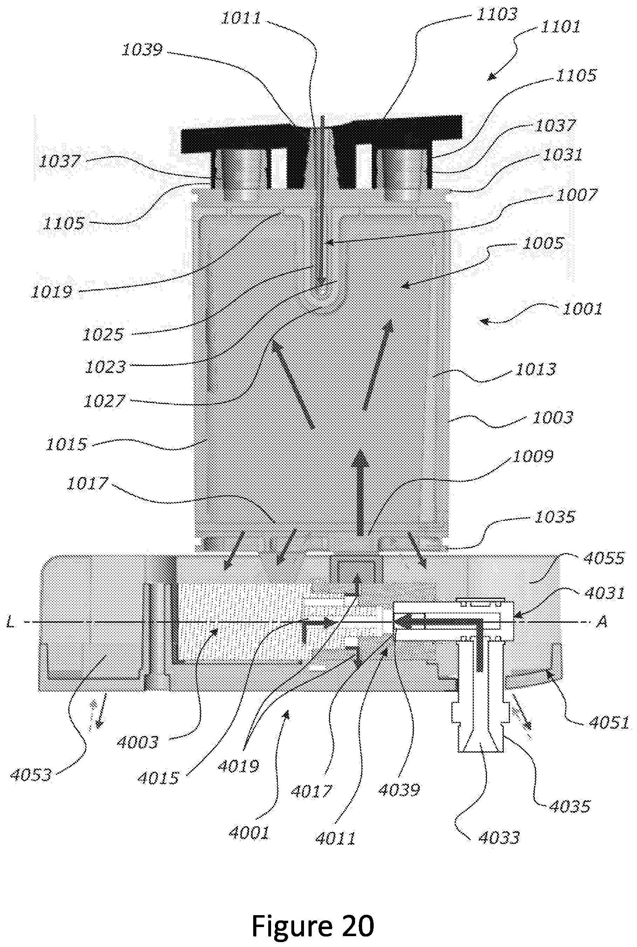

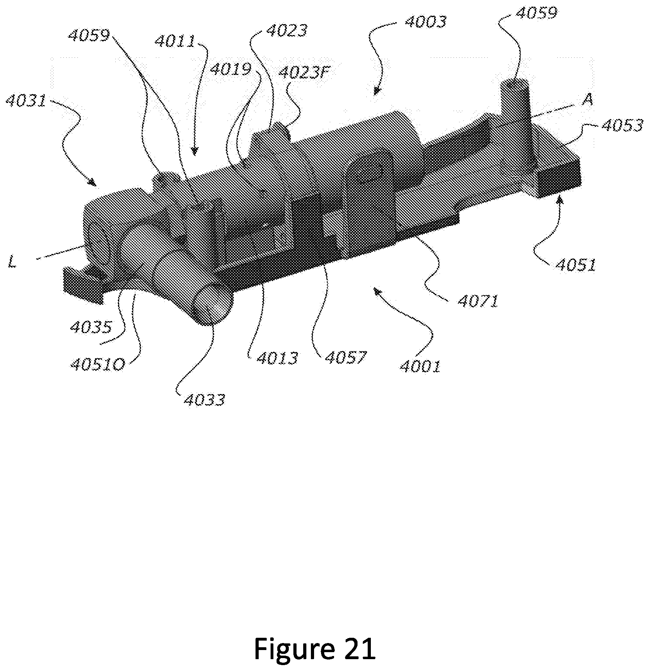

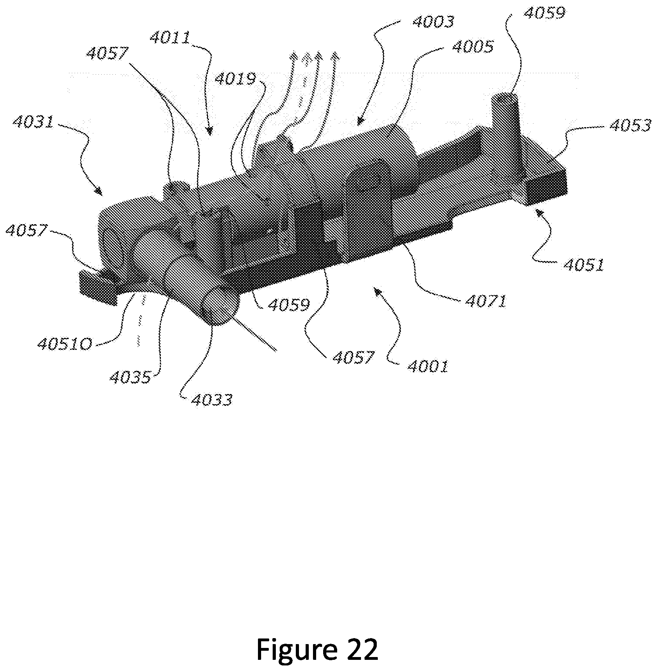

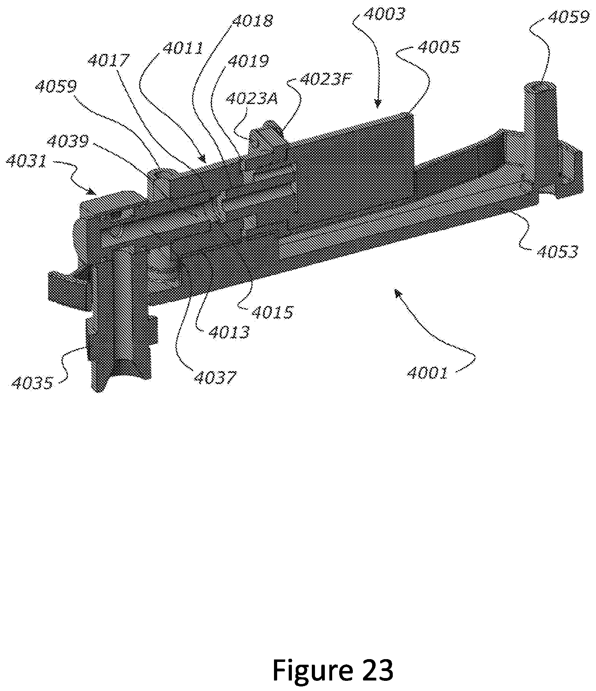

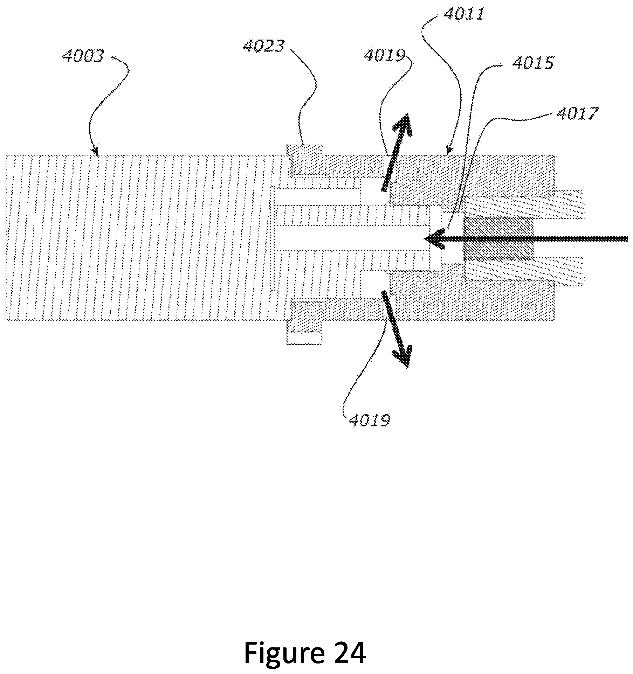

Figures 17 to 24 show a first configuration of a valve module 4001. The valve module 4001 controls the flow of oxygen and/or other gases entering the gas flow path of the apparatus 10, and enables the apparatus 10 to regulate the proportion of oxygen entrained in the airflow. The valve module is formed as a modular unit for ease of manufacture, assembly, servicing, or replacement, for example in the event of malfunction, routine maintenance, or future upgrade/improvement.

The valve module 4001 inserts vertically in an upward direction into the valve module receptacle 306 in the lower chassis 202 of the main housing. In alternative configurations, the valve module may be insertable in a different direction into the housing, such as a forward direction, downward direction, rearward direction, or side direction. The valve module 4001 is removably engageable with the main housing of the apparatus, such that the valve module 4001 is substantially received in the housing and is accessible from the exterior of the housing. In some configurations, the valve module 4001 can be fixed within the main hosing and not removable. Part of the valve module 4001 is arranged to be substantially flush with an external wall of the housing when the valve module is removably engaged with the housing.

Because the valve module is modular and is accessible from the exterior of the housing, the valve module can be replaced without significant disassembly of the apparatus 10 and without compromising seals of the housing of the apparatus. Because the valve module 4001 is substantially received within the housing, when the valve module is engaged with the housing it becomes integrated with the housing and does not increase the size or bulk of the housing. Additionally, the components of the valve module such as the valve 4003 and valve manifold 4011 described below are protected in use because they are positioned within the valve carrier 4051 and main housing of the apparatus in use. This configuration significantly reduces the likelihood of damage of the valve module and valve module components if the apparatus 10 is inadvertently knocked or dropped.

The valve module comprises a flow control valve 4003 that is arranged to control a flow of gas through a valve manifold 4011. The valve is arranged to control a flow of gas into part of the apparatus. For example, the valve may be arranged to control a flow of gas to a filter module 1001. Alternatively, the valve 4003 may be arranged to control a flow of gas to another part of the apparatus. The valve module 4001 and filter module 1001 are positioned upstream of the blower 402 and motor and/or sensor module 400. In some embodiments, the valve module 4001 and filter module 1001are positioned downstream of the blower 402.

The valve 4003 comprises a cylindrical body 4005 and a valve member in the body.

The flow control valve could be a solenoid valve, could be motor-driven, or could be piezo-operated for example.

In a solenoid valve, the valve member is actuated between open and closed positions. The solenoid valve may be a proportional valve. The extent of gas flow through the valve (i.e. due to the size of the valve opening) is relative to the electrical current supplied to the valve.

Alternatively, the solenoid valve may be controlled with a modulated input signal, so that the valve is modulated between open and closed positions.

The valve 4003 could be a needle valve, plunger valve, gate valve, ball valve, butterfly valve, globe valve, etc. The valve may be of the pressure compensated type.

In some configurations, the valve is a normally-closed valve; that is, the valve is closed when powered off. That will prevent a connected gas supply line continuously releasing oxygen or other gas when the apparatus is powered off. In some alternative configurations, the valve is a normally-open valve.

In some configurations, the valve 4003 is an electrically actuated proportional solenoid valve. For example, the valve may be a µProp valve available from Staiger GmbH & Co. KG of Erligheim, Germany, may be an Asco 202 series Preciflow valve available from Emerson/Asco Valves of New Jersey, or may be any other suitable type of valve.

The valve may have a coaxial inlet-outlet configuration.

The valve module 4001 comprises a valve manifold 4011 which has a body 4013 defining a gas flow path 4015 between a valve manifold gases inlet 4017 and one or more valve manifold gases outlets 4019. The gases inlet 4017 of the valve manifold is axially located at or toward an end of the valve manifold. In some configurations the valve manifold 4011 has a single gases outlet 4019, which is radially located on the valve manifold. In some configurations, the valve manifold 4011 comprises a plurality of valve manifold gases outlets 4019 that are radially located about the valve manifold. The valve manifold outlets 4019 are arranged to deliver gases from the valve manifold gases inlet 4017 to a gases inlet of the filter module 1001. The radial arrangement of outlet(s) 4019 assists with directing oxygen (or other gas) towards the filter module, minimizing loss of oxygen and enhancing entrainment efficiency. The valve 4003 is arranged to control a flow of gas from the valve manifold gases inlet 4017 to the valve manifold gases outlet(s) 4019. When the valve is 'closed', gas flow from the gases inlet 4017 to the gases outlet(s) 4019 is prevented. When the valve is 'open', gas flow from the gases inlet 4017 to the gases outlet(s) 4019 is enabled.

An end 4018 of the valve manifold 4011 opposite to the gases inlet receives and sealingly engages with the valve 4003 such that the valve and valve manifold are in fluid communication. The end 4018 comprises a flange 4023 to mount to the valve. The flange 4023 has apertures 4023A to receive fasteners 4023F to fasten the manifold to the valve 4003. O-ring(s) may be provided about the periphery of the interface between the valve 4003 and the valve manifold 4011 to sealingly engage the valve with the valve manifold.

The valve manifold 4011 directs/disperses oxygen from the valve via radially located gases outlets 4019. In some embodiments, a single gases outlet 4019 is provided in the valve manifold. As oxygen passes through the outlet(s), noise is generated. Because the apparatus may be used in medical and/or home environments in close proximity to the patient, it is desirable to minimize the noise produced.

Additionally, or alternatively, a hood, duct, or channel may be formed around, in proximity to, or in fluid communication with the valve manifold outlet(s) 4019 in order to reduce noise. Additionally and/or alternatively, foam, or the like, may be placed around the valve manifold, in proximity to the valve manifold outlets, to reduce noise.

A small filter may be provided inside the valve manifold gases inlet 4017 inlet to prevent the introduction of dust or particulates into the valve.

An end of the valve manifold corresponding to the gases inlet 4015 is arranged to receive and connect to a connector 4031. In the form shown, the connector 4031 is a swivel connector. Alternatively, the connector 4031 may be arranged such that a gases inlet 4033 of the connector can move in a different way, such as a translational movement or pivoting movement for example.

The valve module 4001 is located at the start of the flow path of the apparatus. If the valve 4003 was to be obstructed (i.e. by dust, particulate, etc.) such that it would be held open, excess pressurized oxygen or other gas would 'dump' out ambient air entry opening(s) in the valve carrier 4051 (e.g. the opening shown beneath the swivel connector in figure 26). This would prevent any excess pressure reaching the patient. As such, the system may be considered inherently pressure limited without the use of a pressure relief valve.

Opening(s) 4051O are provided in the valve carrier 4051 to allow ambient air to be drawn in to the gas flow path of the apparatus. The ambient air flow path passes near or adjacent to the valve. In the form shown, the opening 4051O is located around the gases inlet of the swivel connector. Additionally, or alternatively, the opening may be located elsewhere in the valve carrier. When the blower motor 402 of the apparatus is operated, that will create suction through the filter module and valve module, to suck ambient air into the apparatus. The ambient air flow path passes through the valve module and allows ambient air to be entrained with the flow of gas from the flow control valve. The ambient air flow path has a gas outlet adapted to deliver ambient air such that it flows past one or more temperature sensors of the apparatus for delivering a flow of gas.

The apparatus may simultaneously draw in gas from the gases inlet of the valve manifold and ambient air, or the pressurization of gas from the gases inlet may force that gas through the filter. The gases will exit the valve module and enter the gases inlets in the filter. The apparatus may be configured such that the gas from the gases inlet and the ambient air are dynamically entrained/mixed in the apparatus prior to being delivered to the gases outlet of the apparatus.

The valve module may be configured to minimize pressure drop across the valve module by having one or more of: the large opening 4051O for ambient air located around the swivel connector and/or elsewhere; radiuses/rounded/-sloped edges in the flow path (i.e. inside the valve manifold, for example) to minimize turbulence and smooth flow.

This valve module 4001 described herein are arranged to directly couple with the filter 1001 to provide a gas flow path from the valve module to the filter. A hose connection is not required between the valve module and the filter module. This minimizes the size of the components and makes it easy to connect and disconnect the modular valve module and filter module.

The filter modules and valve modules described herein may provide varying gas flow paths for the apparatus. For example, the valve module may control the flow of oxygen entering the gas flow path of the apparatus, via the valve module and filter module. Alternatively, the valve module may be bypassed by means of direct connection of an alternative oxygen source to the filter module by the first sub-compartment gases inlet (inlet 1011 of figure 37 for example). This may be practical in circumstances where a user may wish to manually adjust the oxygen supply (i.e. such as by the wall supply rotameter).

It will be appreciated that the filter modules and the valve modules described herein may be used separately in apparatuses for delivering a flow of gas. Alternatively, the filter and the valve module may be used together as a filer and valve assembly for improved functionality.

In the configurations shown, the apparatus 10 receives oxygen by at least one of the following:

via the valve module (for automatic oxygen regulation by the apparatus), or

via the alternative gases inlet provided on the top of the filter (allowing attachment of a manually adjustable oxygen supply - i.e. such as by the wall supply rotameter).

The various configurations described are exemplary configurations only. Any one or more features from any of the configurations may be used in combination with any one or more features from any of the other configurations.

For example, the swivel connector used in the valve module may have additional functionality. In some configurations, the swivel connector may be arranged to swivel about more than one axis; and may for example have two adjacent swivel connection portions with swivel axes that are transverse to each other, so that the gases inlet of the swivel connector can rotate around the two axes. In some configurations, the swivel connector may comprise a ball and socket arrangement or similar, to enable the gases inlet of the swivel connector to rotate in substantially any direction. In some configurations, the swivel connector may be arranged to provide both swiveling and translational movement; so that the gases inlet of the swivel connector may both swivel about one or more axes and may also travel linearly for example. This may be practical for translating the gases inlet from one portion of the apparatus to another, such as from one side of the apparatus to the other of the apparatus for example. In some configurations, the gases inlet may be arranged to translate instead of rotate.

As another example, while the motor and/or sensor sub-assembly recess is described as being in the underside of the main housing, it could alternatively be in a rear, side, front, or top of the housing. With such a variant, the air and/or oxygen inlets may also be positioned differently as required.

As another example, rather than the liquid chamber and chamber bay being configured so that the liquid chamber is inserted into and removed from the chamber bay from a front of the housing, the configuration could be such that the liquid chamber is inserted into and removed from the chamber bay from a side, rear, or top of the housing.

As another example, while the filter modules are described as being inserted into the housing from above and the valve modules inserted into the housing from below, either or both of those components could be inserted into any suitable part of the housing, such as an upper part, lower part, side part, front part, or rear part.

The filter module and valve module are described with reference to a flow therapy apparatus that is capable of delivering heated and humidified gases to a patient or user. The apparatus may be suitable for treating chronic obstructive pulmonary disease (COPD). The apparatus may be configured to deliver gases to a patient interface at a high flow rate (high flow therapy), particularly nasal high flow therapy.

Alternatively, the filter module and/or valve module may be used in an apparatus for a different purpose. The apparatus may be a high flow therapy apparatus, or may be a low flow therapy apparatus. The features may also be provided in an apparatus for providing continuous positive airway pressure (CPAP), which may deliver gases (humidified or otherwise) at positive pressure.

The filter module and/or valve module may alternatively be used with an apparatus that does not require a humidifier and therefore does not require the liquid chamber 300 or chamber bay 108 features. For example, it will be appreciated that the configuration that isolates the motor and gas flow path from the electrical and electronic components has broad applications in other types of gas delivery apparatuses.

The 'flow therapy apparatus' language is intended to cover all such variants.

## Claims

1. A respiratory assistance apparatus (1) comprising:

    a flow generator (10) configured to provide breathing gases (922) to a patient, the breathing gases (922) comprising supplemental oxygen provided from an oxygen source,
    a controller (13) configured to control an oxygen concentration of the breathing gases (922),
    at least one sensor, the at least one sensor configured to provide a measurement indicative of a patient's blood oxygen concentration to the controller (13),
    wherein the controller (13) is configured to determine a target oxygen concentration of the breathing gases (922),
    **characterized in that**
    the controller (13) is configured to calculate an estimated future value of the patient's blood oxygen concentration based on:
    a difference between an initial oxygen concentration (930) of the breathing gases (922) and the target oxygen concentration of the breathing gases (922), and
    the measurement indicative of the patient's blood oxygen concentration.

2. The respiratory assistance apparatus (1) of claim 1, wherein the controller (13) is configured to control the oxygen concentration of the breathing gases (922) based on the target oxygen concentration.

3. The respiratory assistance apparatus (1) of claim 1 or claim 2, wherein the estimated future value of the patient's blood oxygen concentration is further based on a time from when the controller (13) controls the oxygen concentration of the breathing gases (922) to the target oxygen concentration.

4. The respiratory assistance apparatus (1) of any one of claims 1 to 3, wherein the controller (13) is configured to compare said estimated future value of the patient's blood oxygen concentration with a blood oxygen concentration alarm range, and generate an alarm output if the estimated future value of the patient's blood oxygen concentration is not within said blood oxygen concentration alarm range, and wherein the blood oxygen concentration alarm range comprises an upper blood oxygen concentration alarm threshold, and a lower blood oxygen concentration alarm threshold.

5. The respiratory assistance apparatus (1) of claim 4, wherein the controller (13) is configured to generate an alarm output based on the estimated future value of the patient's blood oxygen concentration and a comparison with a blood oxygen concentration alarm threshold, and wherein the controller (13) is configured to generate said alarm output if the estimated future value of the patient's blood oxygen concentration is above an upper blood oxygen concentration alarm threshold or below a lower blood oxygen concentration alarm threshold.

6. The respiratory assistance apparatus (1) of claim 4 or claim 5, wherein the upper blood oxygen concentration alarm threshold and/or the lower blood oxygen concentration alarm threshold is/are determined based on a time from when the controller (13) controls the oxygen concentration of the breathing gases (922) to the target oxygen concentration.

7. The respiratory assistance apparatus (1) of any one of claims 4 to 6, wherein the upper blood oxygen concentration alarm threshold and/or the lower blood oxygen concentration alarm threshold is/are determined based on an amount of a change in target oxygen concentration.

8. The respiratory assistance apparatus (1) of any one of claims 4 to 6, wherein the upper blood oxygen concentration alarm threshold and/or the lower blood oxygen concentration alarm threshold is/are determined based on the target oxygen concentration

9. The respiratory assistance apparatus (1) of any one of claims 4 to 8, wherein the upper blood oxygen concentration alarm threshold and/or the lower blood oxygen concentration alarm threshold is/are determined based on the current oxygen concentration of gases.

10. The respiratory assistance apparatus (1) of any one of claims 4 to 9, wherein the upper blood oxygen concentration alarm threshold and/or the lower blood oxygen concentration alarm threshold is/are based on a target blood oxygen concentration.

11. The respiratory assistance apparatus (1) of any one of claims 1 to 10, wherein the controller (13) is configured to receive an input, and based on the input determine the target oxygen concentration of the breathing gases (922).

12. The respiratory assistance apparatus (1) of claim 11, wherein the input comprises a target oxygen concentration range comprising an upper target oxygen concentration, and a lower target oxygen concentration.

13. The respiratory assistance apparatus (1) of claim 12, wherein the upper blood oxygen concentration alarm threshold is based on the difference between the target oxygen concentration and the upper target oxygen concentration.

14. The respiratory assistance apparatus (1) of any one of claims 1 to 13, wherein the estimated future value of the patient's blood oxygen concentration is based on an oxygen efficiency of the patient and/or a haemoglobin saturation function and/or a haemoglobin saturation function.

15. The respiratory assistance apparatus (1) of claim 14, wherein the oxygen efficiency of the patient is based on the measurement indicative of a patient's blood oxygen concentration divided by a measured oxygen concentration of the breathing gases (922), and/or received as an input.

**Patentansprüche**

1. Atmungsunterstützungseinrichtung (1), umfassend:

einen Strömungsgenerator (10), der dazu konfiguriert ist, einem Patienten Atemgase (922) bereitzustellen, wobei die Atemgase (922) zusätzlichen Sauerstoff umfassen, der von einer Sauerstoffquelle bereitgestellt wird, eine Steuereinheit (13), die dazu konfiguriert ist, eine Sauerstoffkonzentration der Atemgase (922) zu steuern, mindestens einen Sensor, wobei der mindestens eine Sensor dazu konfiguriert ist, der Steuereinheit (13) ein Messergebnis bereitzustellen, das die Blutsauerstoffkonzentration eines Patienten angibt, wobei die Steuereinheit (13) dazu konfiguriert ist, eine Ziel-Sauerstoffkonzentration der Atemgase (922) zu bestimmen, **dadurch gekennzeichnet, dass** die Steuereinheit (13) dazu konfiguriert ist, einen geschätzten zukünftigen Wert der Blutsauerstoffkonzentration des Patienten zu berechnen, basierend auf:

einer Differenz zwischen einer anfänglichen Sauerstoffkonzentration (930) der Atemgase (922) und der Ziel-Sauerstoffkonzentration der Atemgase (922), und dem Messergebnis, das die Blutsauerstoffkonzentration des Patienten angibt.

2. Atmungsunterstützungseinrichtung (1) nach Anspruch 1, wobei die Steuereinheit (13) dazu konfiguriert ist, die Sauerstoffkonzentration der Atemgase (922) basierend auf der Ziel-Sauerstoffkonzentration zu steuern.

3.  Atmungsunterstützungseinrichtung (1) nach Anspruch 1 oder Anspruch 2, wobei der geschätzte zukünftige Wert der Blutsauerstoffkonzentration des Patienten weiter auf einer Zeit basiert, ab der die Steuereinheit (13) die Sauerstoffkonzentration der Atemgase (922) zu der Ziel-Sauerstoffkonzentration steuert.

4.  Atmungsunterstützungseinrichtung (1) nach einem der Ansprüche 1 bis 3, wobei die Steuereinheit (13) dazu konfiguriert ist, den geschätzten zukünftigen Wert der Blutsauerstoffkonzentration des Patienten mit einem Alarmbereich der Blutsauerstoffkonzentration zu vergleichen, und eine Alarmausgabe zu erzeugen, wenn der geschätzte zukünftige Wert der Blutsauerstoffkonzentration des Patienten nicht innerhalb des Alarmbereichs der Blutsauerstoffkonzentration liegt, und wobei der Alarmbereich der Blutsauerstoffkonzentration einen oberen Alarmschwellenwert der Blutsauerstoffkonzentration und einen unteren Alarmschwellenwert der Blutsauerstoffkonzentration umfasst.

5.  Atmungsunterstützungseinrichtung (1) nach Anspruch 4, wobei die Steuereinheit (13) dazu konfiguriert ist, eine Alarmausgabe basierend auf dem geschätzten zukünftigen Wert der Blutsauerstoffkonzentration des Patienten und einem Vergleich mit einem Alarmschwellenwert der Blutsauerstoffkonzentration zu erzeugen, und wobei die Steuereinheit (13) dazu konfiguriert ist, die Alarmausgabe zu erzeugen, wenn der geschätzte zukünftige Wert der Blutsauerstoffkonzentration des Patienten über einem oberen Alarmschwellenwert der Blutsauerstoffkonzentration oder unter einem unteren Alarmschwellenwert der Blutsauerstoffkonzentration liegt.

6.  Atmungsunterstützungseinrichtung (1) nach Anspruch 4 oder Anspruch 5, wobei der obere Alarmschwellenwert der Blutsauerstoffkonzentration und/oder der untere Alarmschwellenwert der Blutsauerstoffkonzentration basierend auf einer Zeit, ab der die Steuereinheit (13) die Sauerstoffkonzentration der Atemgase (922) zu der Ziel-Sauerstoffkonzentration steuert, bestimmt ist/sind.

7.  Atmungsunterstützungseinrichtung (1) nach einem der Ansprüche 4 bis 6, wobei der obere Alarmschwellenwert der Blutsauerstoffkonzentration und/oder der untere Alarmschwellenwert der Blutsauerstoffkonzentration basierend auf einem Betrag einer Änderung in der Ziel-Sauerstoffkonzentration bestimmt ist/sind.

8.  Atmungsunterstützungseinrichtung (1) nach einem der Ansprüche 4 bis 6, wobei der obere Alarmschwellenwert der Blutsauerstoffkonzentration und/oder der untere Alarmschwellenwert der Blutsauerstoffkonzentration basierend auf der Ziel-Sauerstoffkonzentration bestimmt ist/sind

9.  Atmungsunterstützungseinrichtung (1) nach einem der Ansprüche 4 bis 8, wobei der obere Alarmschwellenwert der Blutsauerstoffkonzentration und/oder der untere Alarmschwellenwert der Blutsauerstoffkonzentration basierend auf der aktuellen Sauerstoffkonzentration von Gasen bestimmt ist/sind.

10. Atmungsunterstützungseinrichtung (1) nach einem der Ansprüche 4 bis 9, wobei der obere Alarmschwellenwert der Blutsauerstoffkonzentration und/oder der untere Alarmschwellenwert der Blutsauerstoffkonzentration auf einer Ziel-Blutsauerstoffkonzentration basiert/basieren.

11. Atmungsunterstützungseinrichtung (1) nach einem der Ansprüche 1 bis 10, wobei die Steuereinheit (13) dazu konfiguriert ist, eine Eingabe zu empfangen, und basierend auf der Eingabe die Ziel-Sauerstoffkonzentration der Atemgase (922) zu bestimmen.

12. Atmungsunterstützungseinrichtung (1) nach Anspruch 11, wobei die Eingabe einen Ziel-Sauerstoffkonzentrationsbereich umfasst, der eine obere Ziel-Sauerstoffkonzentration und eine untere Ziel-Sauerstoffkonzentration umfasst.

13. Atmungsunterstützungseinrichtung (1) nach Anspruch 12, wobei der obere Alarmschwellenwert der Blutsauerstoffkonzentration auf der Differenz zwischen der Ziel-Sauerstoffkonzentration und der oberen Ziel-Sauerstoffkonzentration basiert.

14. Atmungsunterstützungseinrichtung (1) nach einem der Ansprüche 1 bis 13, der geschätzte zukünftige Wert der Blutsauerstoffkonzentration des Patienten auf einer Sauerstoffeffizienz des Patienten und/oder einer Hämoglobinsättigungsfunktion und/oder einer Hämoglobinsättigungsfunktion basiert.

15. Atmungsunterstützungseinrichtung (1) nach Anspruch 14, wobei die Sauerstoffeffizienz des Patienten auf dem Messergebnis, das die Blutsauerstoffkonzentration des Patienten angibt, dividiert durch eine gemessene Sauerstoffkonzentration der Atemgase (922) basiert, und/oder als eine Eingabe empfangen wird.

**Revendications**

1.  Appareil (1) d'assistance respiratoire comprenant :

    un générateur (10) de débit configuré pour fournir des gaz respiratoires (922) à un patient, les gaz respiratoires (922) comprenant de l'oxygène supplémentaire fourni à partir d'une source d'oxygène, un dispositif (13) de commande configuré pour commander une concentration d'oxygène des gaz respiratoires (922), au moins un capteur, l'au moins un capteur étant configuré pour fournir une mesure indicative de la concentration d'oxygène dans le sang d'un patient au dispositif (13) de commande,
    dans lequel le dispositif (13) de commande est configuré pour déterminer une concentration cible en oxygène des gaz respiratoires (922),
    **caractérisé en ce que**
    le dispositif (13) de commande est configuré pour calculer une valeur future estimée de la concentration d'oxygène dans le sang du patient sur la base :

    d'une différence entre une concentration d'oxygène initiale (930) des gaz respiratoires (922) et la concentration d'oxygène cible des gaz respiratoires (922), et
    de la mesure indiquant la concentration d'oxygène dans le sang du patient.

2.  Appareil (1) d'assistance respiratoire selon la revendication 1, dans lequel le dispositif (13) de commande est configuré pour commander la concentration d'oxygène des gaz respiratoires (922) sur la base de la concentration d'oxygène cible.

3.  Appareil (1) d'assistance respiratoire selon la revendication 1 ou la revendication 2, dans lequel la valeur future estimée de la concentration d'oxygène dans le sang du patient est en outre basée sur un moment à partir duquel le dispositif (13) de commande commande la concentration d'oxygène des gaz respiratoires (922) jusqu'à la concentration d'oxygène cible.

4.  Appareil (1) d'assistance respiratoire selon l'une quelconque des revendications 1 à 3, dans lequel le dispositif (13) de commande est configuré pour comparer ladite valeur future estimée de la concentration d'oxygène dans le sang du patient à une plage d'alarme de concentration d'oxygène dans le sang, et générer une sortie d'alarme si la valeur future estimée de la concentration d'oxygène dans le sang du patient n'est pas dans ladite plage d'alarme de concentration d'oxygène dans le sang, et dans lequel la plage d'alarme de concentration d'oxygène dans le sang comprend un seuil d'alarme de concentration d'oxygène dans le sang supérieur et un seuil d'alarme de concentration d'oxygène dans le sang inférieur.

5.  Appareil (1) d'assistance respiratoire selon la revendication 4, dans lequel le dispositif (13) de commande est configuré pour générer une sortie d'alarme sur la base de la valeur future estimée de la concentration d'oxygène dans le sang du patient et d'une comparaison avec un seuil d'alarme de concentration d'oxygène dans le sang, et dans lequel le dispositif (13) de commande est configuré pour générer ladite sortie d'alarme si la valeur future estimée de la concentration d'oxygène dans le sang du patient est supérieure à un seuil d'alarme de concentration d'oxygène dans le sang supérieur ou inférieure à un seuil d'alarme de concentration d'oxygène dans le sang inférieur.

6.  Appareil (1) d'assistance respiratoire selon la revendication 4 ou la revendication 5, dans lequel le seuil d'alarme de concentration d'oxygène dans le sang supérieur et/ou le seuil d'alarme de concentration d'oxygène dans le sang inférieur est/sont déterminé(s) sur la base d'un moment à partir duquel le dispositif (13) de commande commande la concentration d'oxygène des gaz respiratoires (922) jusqu'à la concentration d'oxygène cible.

7.  Appareil (1) d'assistance respiratoire selon l'une quelconque des revendications 4 à 6, dans lequel le seuil d'alarme de concentration d'oxygène dans le sang supérieur et/ou le seuil d'alarme de concentration d'oxygène dans le sang inférieur est/sont déterminé(s) sur la base d'une quantité d'un changement de concentration d'oxygène cible.

8.  Appareil (1) d'assistance respiratoire selon l'une quelconque des revendications 4 à 6, dans lequel le seuil d'alarme de concentration d'oxygène dans le sang supérieur et/ou le seuil d'alarme de concentration d'oxygène dans le sang inférieur est/sont déterminé(s) sur la base de la concentration d'oxygène cible.

9.  Appareil (1) d'assistance respiratoire selon l'une quelconque des revendications 4 à 8, dans lequel le seuil d'alarme de concentration d'oxygène dans le sang supérieur et/ou le seuil d'alarme de concentration d'oxygène dans le sang inférieur est/sont déterminé(s) sur la base de la concentration d'oxygène actuelle des gaz.

10. Appareil (1) d'assistance respiratoire selon l'une quelconque des revendications 4 à 9, dans lequel le seuil d'alarme de concentration d'oxygène dans le sang supérieur et/ou le seuil d'alarme de concentration d'oxygène dans le sang inférieur est/sont basé(s) sur une concentration d'oxygène dans le sang cible.

11. Appareil (1) d'assistance respiratoire selon l'une quelconque des revendications 1 à 10, dans lequel le dispositif (13) de commande est configuré pour recevoir une entrée, et en fonction de l'entrée, déterminer la concentration d'oxygène cible des gaz respiratoires (922).

12. Appareil (1) d'assistance respiratoire selon la revendication 11, dans lequel l'entrée comprend une plage de concentration d'oxygène cible comprenant une concentration d'oxygène cible supérieure et une concentration d'oxygène cible inférieure.

13. Appareil (1) d'assistance respiratoire selon la revendication 12, dans lequel le seuil d'alarme de concentration d'oxygène dans le sang supérieur est basé sur la différence entre la concentration d'oxygène cible et la concentration d'oxygène cible supérieure.

14. Appareil (1) d'assistance respiratoire selon l'une quelconque des revendications 1 à 13, dans lequel la valeur future estimée de la concentration d'oxygène dans le sang du patient est basée sur une efficacité en oxygène du patient et/ou une fonction de saturation en hémoglobine et/ou une fonction de saturation en hémoglobine.

15. Appareil (1) d'assistance respiratoire selon la revendication 14, dans lequel l'efficacité en oxygène du patient est basée sur la mesure indicative de la concentration d'oxygène dans le sang d'un patient divisée par une concentration d'oxygène mesurée des gaz respiratoires (922) et/ou reçue en tant qu'entrée.

Figure 1A

EP 4 048 356 B1

Figure 1B

Figure 1E

2360

2364 RX

2306
2308

2366

2362 TX

Figure 1F

2370

2374 TX/RX

2306
2308

2376

2372 TX/RX

Figure 1C

2300

2306
2308

RX 2304

2310

2302 TX

Figure 1D

2320

2306
2308

TX/RX 2324

2310

2322 TX/RX

Oxygen concentration of breathing gases over time

Estimated blood oxygen concentration over time

Figure 2A

Figure 2B

Figure 3

```
┌─────────────────────────────────┐
│    DETERMINE INITIAL OXYGEN     │ ⌐ 901
│       CONCENTRATION OF          │
│       BREATHING GASES           │
└─────────────────────────────────┘
               │
               ▼
┌─────────────────────────────────┐
│   DETERMINE ADJUSTED OXYGEN     │ ⌐ 902
│       CONCENTRATION OF          │
│       BREATHING GASES           │
└─────────────────────────────────┘
               │
               ▼
┌─────────────────────────────────┐
│      DETERMINE PATIENT'S        │ ⌐ 903
│        BLOOD OXYGEN             │
│        CONCENTRATION            │
└─────────────────────────────────┘
               │
               ▼
┌─────────────────────────────────┐
│    ESTIMATE FUTURE VALUE OF     │ ⌐ 904
│    PATIENT'S BLOOD OXYGEN       │
│        CONCENTRATION            │
└─────────────────────────────────┘
```

# Figure 4A

UPDATE STORED OXYGEN
CONCENTRATION DATA — 911

DETERMINE PATIENT'S
BLOOD OXYGEN
CONCENTRATION — 912

ESTIMATE FUTURE VALUE OF
PATIENT'S BLOOD OXYGEN
CONCENTRATION — 913

# Figure 4B

EXECUTE ESTIMATE
UPDATE PHASE — 916

DETERMINE PATIENT'S
BLOOD OXYGEN
CONCENTRATION — 917

ESTIMATE FUTURE VALUE OF
PATIENT'S BLOOD OXYGEN
CONCENTRATION — 918

# Figure 4C

916

APPLY DECAY FACTOR
TO SUM OF CHANGES
990

DETERMINE OXYGEN
CONCENTRATION OF
BREATHING GASES
991

DETERMINE OXYGEN
CONCENTRATION OF BREATHING
GASES AT PREVIOUS ESTIMATE
992

CALCULATE OXYGEN
CONCENTRATION DIFFERENCE
993

UPDATE SUM
OF CHANGES
994

Figure 4D

Estimated blood oxygen concentration over time

996

997

t₁    t₂              t₃    Time

# Figure 5A

Estimated blood oxygen concentration over time

998

t₁    t₂              t₃    Time

# Figure 5B

Figure 6

Oxygen concentration of breathing gases over time

Figure 7

Oxygen concentration of breathing gases over time

Figure 8A

Blood oxygen concentration over time

Figure 8B

```
┌─────────────────────────────┐ ⌐960
│    UPDATE STORE OXYGEN      │
│    CONCENTRATION DATA       │◄─┐
└─────────────────────────────┘  │
              │                  │
              ▼                  │
┌─────────────────────────────┐ ⌐961
│     DETERMINE PATIENT'S     │  │
│       BLOOD OXYGEN          │  │
│      CONCENTRATION          │  │
└─────────────────────────────┘  │
              │                  │
              ▼                  │
┌─────────────────────────────┐ ⌐962
│     DETERMINE TARGET        │
│   OXYGEN CONCENTRATION      │
│  RANGE OF BREATHING GASES   │
└─────────────────────────────┘
              │
              ▼
┌─────────────────────────────┐ ⌐963
│    ESTIMATE MAX OR MIN      │
│   FUTURE VALUE OF PATIENT   │
│ BLOOD OXYGEN CONCENTRATION  │
└─────────────────────────────┘
```

# Figure 9A

```
┌─────────────────────────────┐
│     EXECUTE ESTIMATE        │── 970
│     UPDATE PHASE            │◀┐
└─────────────┬───────────────┘ │
              │                 │
              ▼                 │
┌─────────────────────────────┐ │
│    DETERMINE PATIENT'S      │── 971
│    BLOOD OXYGEN             │
│    CONCENTRATION            │
└─────────────┬───────────────┘
              │
              ▼
┌─────────────────────────────┐
│    DETERMINE TARGET         │── 972
│    OXYGEN CONCENTRATION     │
│  RANGE OF BREATHING GASES   │
└─────────────┬───────────────┘
              │
              ▼
┌─────────────────────────────┐
│    ESTIMATE MAX OR MIN      │── 973
│  FUTURE VALUE OF PATIENT    │
│ BLOOD OXYGEN CONCENTRATION  │
└─────────────────────────────┘
```

## Figure 9B

Oxygen concentration of breathing gases over time

Figure 10

Figure 10A

Figure 11

Figure 12

Figure 13

Figure 14

Figure 15

Figure 16

Figure 17A

Figure 17B

Figure 18

Figure 19

Figure 20

Figure 21

Figure 22

Figure 23

Figure 24

1

Respiratory assistance
apparatus

2

Auxiliary device

Display

Controller

Figure 25

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019070136 A1
- US 62409543
- US 62488841
- NZ 2016050193 W
- WO IB2016053761 A
- NZ 2018050137 W